# EUROPEAN PATENT SPECIFICATION

(11) **EP 3 564 267 B1**
(45) Date of publication and mention of the grant of the patent: **17.03.2021**
(21) Application number: 19162362.8
(22) Date of filing: 14.06.2007
(51) Int. Cl.: C07K 19/00, C12N 15/62

(54) **PROTEOLYTICALLY CLEAVABLE FUSION PROTEINS WITH HIGH MOLAR SPECIFIC ACTIVITY**
PROTEOLYTISCH SPALTBARE FUSIONSPROTEINE MIT HOHER MOLSPEZIFISCHER AKTIVITÄT
PROTÉINES DE FUSION CLIVABLE DE MANIÈRE PROTÉOLYTIQUE AYANT UNE ACTIVITÉ MOLAIRE SPÉCIFIQUE ÉLEVÉE

(30) Priority: 14.06.2006 EP 06012262; 11.07.2006 US 81962006 P
(43) Date of publication of application: 06.11.2019
(62) Divisional of application: 10168453.8
(73) Proprietor: CSL Behring GmbH, 35041 Marburg (DE)
(72) Inventor: METZNER, Hubert, 35041 Marburg (DE); WEIMER, Thomas, 35075 Gladenbach (DE); SCHULTE, Stefan, 35043 Marburg (DE)
(74) Representative: Wise, Daniel Joseph

(56) References cited:
- EP-A- 1 444 986
- EP-A1- 1 816 201
- WO-A-96/01653
- WO-A2-2004/021861
- WO-A2-2004/101739
- WO-A2-2004/101740
- SHEFFIELD W P ET AL: "Effects of genetic fusion of factor IX to albumin on in vivo clearance in mice and rabbits", BRITISH JOURNAL OF HAEMATOLOGY, OXFORD, GB, vol. 126, no. 4, August 2004 (2004-08), pages 565-573, XP002395198, ISSN: 0007-1048

### SEQUENCE LISTING

<110> CSL Behring GmbH
<120> Proteolytically cleavable fusion proteins with high molar specific activity
<130> P073645EP
<150> To be assigned
   <151> 2006-06-14
<160> 112
<170> PatentIn version 3.3
<210> 1
   <211> 585
   <212> PRT
   <213> Homo sapiens
<400> 1
<210> 2
   <211> 415
   <212> PRT
   <213> Homo sapiens
<400> 2
   Tyr Asn Ser Gly Lys Leu Glu Glu Phe Val Gln Gly Asn Leu Glu Arg
   1 5 10 15
   Glu Cys Met Glu Glu Lys Cys Ser Phe Glu Glu Ala Arg Glu Val Phe
   20 25 30
   Glu Asn Thr Glu Arg Thr Thr Glu Phe Trp Lys Gln Tyr Val Asp Gly
   35 40 45
   Asp Gln Cys Glu Ser Asn Pro Cys Leu Asn Gly Gly Ser Cys Lys Asp
       50                  55                  60
   Asp Ile Asn Ser Tyr Glu Cys Trp Cys Pro Phe Gly Phe Glu Gly Lys
   65                  70                  75                  80
   Asn Cys Glu Leu Asp Val Thr Cys Asn Ile Lys Asn Gly Arg Cys Glu
                   85                  90                  95
   Gln Phe Cys Lys Asn Ser Ala Asp Asn Lys Val Val Cys Ser Cys Thr
               100                 105                 110
   Glu Gly Tyr Arg Leu Ala Glu Asn Gln Lys Ser Cys Glu Pro Ala Val
           115                 120                 125
   Pro Phe Pro Cys Gly Arg Val Ser Val Ser Gln Thr Ser Lys Leu Thr
       130                 135                 140
   Arg Ala Glu Thr Val Phe Pro Asp Val Asp Tyr Val Asn Ser Thr Glu
   145                 150                 155                 160
   Ala Glu Thr Ile Leu Asp Asn Ile Thr Gln Ser Thr Gln Ser Phe Asn
                   165                 170                 175
   Asp Phe Thr Arg Val Val Gly Gly Glu Asp Ala Lys Pro Gly Gln Phe
               180                 185                 190
   Pro Trp Gln Val Val Leu Asn Gly Lys Val Asp Ala Phe Cys Gly Gly
           195                 200                 205
   Ser Ile Val Asn Glu Lys Trp Ile Val Thr Ala Ala His Cys Val Glu
       210                 215                 220
   Thr Gly Val Lys Ile Thr Val Val Ala Gly Glu His Asn Ile Glu Glu
   225                 230                 235                 240
   Thr Glu His Thr Glu Gln Lys Arg Asn Val Ile Arg Ile Ile Pro His
                   245                 250                 255
   His Asn Tyr Asn Ala Ala Ile Asn Lys Tyr Asn His Asp Ile Ala Leu
               260                 265                 270
   Leu Glu Leu Asp Glu Pro Leu Val Leu Asn Ser Tyr Val Thr Pro Ile
           275                 280                 285
   Cys Ile Ala Asp Lys Glu Tyr Thr Asn Ile Phe Leu Lys Phe Gly Ser
       290                 295                 300
   Gly Tyr Val Ser Gly Trp Gly Arg Val Phe His Lys Gly Arg Ser Ala
   305                 310                 315                 320
   Leu Val Leu Gln Tyr Leu Arg Val Pro Leu Val Asp Arg Ala Thr Cys
                   325                 330                 335
   Leu Arg Ser Thr Lys Phe Thr Ile Tyr Asn Asn Met Phe Cys Ala Gly
               340                 345                 350
   Phe His Glu Gly Gly Arg Asp Ser Cys Gln Gly Asp Ser Gly Gly Pro
           355                 360                 365
   His Val Thr Glu Val Glu Gly Thr Ser Phe Leu Thr Gly Ile Ile Ser
       370                 375                 380
   Trp Gly Glu Glu Cys Ala Met Lys Gly Lys Tyr Gly Ile Tyr Thr Lys
   385                 390                 395                 400
   Val Ser Arg Tyr Val Asn Trp Ile Lys Glu Lys Thr Lys Leu Thr
                   405                 410                 415
<210> 3
   <211> 1386
   <212> DNA
   <213> Homo sapiens
<400> 3
   atgcagcgcg tgaacatgat catggcagaa tcaccaggcc tcatcaccat ctgcctttta      60
   ggatatctac tcagtgctga atgtacagtt tttcttgatc atgaaaacgc caacaaaatt     120
   ctgaatcggc caaagaggta taattcaggt aaattggaag agtttgttca agggaacctt     180
   gagagagaat gtatggaaga aaagtgtagt tttgaagaag cacgagaagt ttttgaaaac     240
   actgaaagaa caactgaatt ttggaagcag tatgttgatg gagatcagtg tgagtccaat     300
   ccatgtttaa atggcggcag ttgcaaggat gacattaatt cctatgaatg ttggtgtccc     360
   tttggatttg aaggaaagaa ctgtgaatta gatgtaacat gtaacattaa gaatggcaga     420
   tgcgagcagt tttgtaaaaa tagtgctgat aacaaggtgg tttgctcctg tactgaggga     480
   tatcgacttg cagaaaacca gaagtcctgt gaaccagcag tgccatttcc atgtggaaga     540
   gtttctgttt cacaaacttc taagctcacc cgtgctgaga ctgtttttcc tgatgtggac     600
   tatgtaaatt ctactgaagc tgaaaccatt ttggataaca tcactcaaag cacccaatca     660
   tttaatgact tcactcgggt tgttggtgga gaagatgcca aaccaggtca attcccttgg     720
   caggttgttt tgaatggtaa agttgatgca ttctgtggag gctctatcgt taatgaaaaa     780
   tggattgtaa ctgctgccca ctgtgttgaa actggtgtta aaattacagt tgtcgcaggt     840
   gaacataata ttgaggagac agaacataca gagcaaaagc gaaatgtgat tcgaattatt     900
   cctcaccaca actacaatgc agctattaat aagtacaacc atgacattgc ccttctggaa     960
   ctggacgaac ccttagtgct aaacagctac gttacaccta tttgcattgc tgacaaggaa    1020
   tacacgaaca tcttcctcaa atttggatct ggctatgtaa gtggctgggg aagagtcttc    1080
   cacaaaggga gatcagcttt agttcttcag taccttagag ttccacttgt tgaccgagcc    1140
   acatgtcttc gatctacaaa gttcaccatc tataacaaca tgttctgtgc tggcttccat    1200
   gaaggaggta gagattcatg tcaaggagat agtgggggac cccatgttac tgaagtggaa    1260
   gggaccagtt tcttaactgg aattattagc tggggtgaag agtgtgcaat gaaaggcaaa    1320
   tatggaatat ataccaaggt atcccggtat gtcaactgga ttaaggaaaa aacaaagctc    1380
   acttaa                                                               1386
<210> 4
   <211> 1830
   <212> DNA
   <213> Homo sapiens
<4> 4
<210> 5
   <211> 21
   <212> DNA
   <213> Artificial Sequence
<220>
   <223> Artificial
<400> 5
   ccactttcac aatctgctag c 21
<210> 6
   <211> 23
   <212> DNA
   <213> Artificial Sequence
<220>
   <223> Artificial
<400> 6
   caattccaat gaattaacct tgg 23
<210> 7
   <211> 21
   <212> DNA
   <213> Artificial Sequence
<220>
   <223> Artificial
<400> 7
   atgcagcgcg tgaacatgat c 21
<210> 8
   <211> 25
   <212> DNA
   <213> Artificial Sequence
<220>
   <223> Artificial
<400> 8
   tcattaagtg agctttgttt tttcc 25
<210> 9
   <211> 21
   <212> DNA
   <213> Artificial Sequence
<220>
   <223> Artificial
<400> 9
   gattcgaatt cgcccttatg c 21
<210> 10
   <211> 32
   <212> DNA
   <213> Artificial Sequence
<220>
   <223> Artificial
<400> 10
   cgctcgaggt gagctttgtt ttttccttaa tc 32
<210> 11
   <211> 52
   <212> DNA
   <213> Artificial Sequence
<220>
   <223> Artificial
<400> 11
   ctcgagcggg ggatctggcg ggtctggagg ctctggaggg tcgggaggct ct 52
<210> 12
   <211> 46
   <212> DNA
   <213> Artificial Sequence
<220>
   <223> Artificial
<400> 12
   ggatccagat cccccagagc ctccagagcc tcccgaccct ccagag 46
<210> 13
   <211> 56
   <212> DNA
   <213> Artificial Sequence
<220>
   <223> Artificial
<400> 13
   ctcgagcaat ggatctggcg ggtctggagg ctctggaggg tcgaatggct ctggag 56
<210> 14
   <211> 64
   <212> DNA
   <213> Artificial Sequence
<220>
   <223> Artificial
<400> 14
   ggatccgttc cctccagacc cgccagatcc cccagagcct ccagagccat tcgaccctcc 60
   agag 64
<210> 15
   <211> 46
   <212> DNA
   <213> Artificial Sequence
<220>
   <223> Artificial
<400> 15
   cctcgagctc tgtgagccag acctccaagc tcaccagggc cgagac 46
<210> 16
   <211> 44
   <212> DNA
   <213> Artificial Sequence
<220>
   <223> Artificial
<400> 16
   gggatccgtc cacatcaggg aagacagtct cggccctggt gagc 44
<210> 17
   <211> 33
   <212> DNA
   <213> Artificial Sequence
<220>
   <223> Artificial
<400> 17
   ggaaaaaaca aagctcactt ctgtgagcca gac 33
<210> 18
   <211> 33
   <212> DNA
   <213> Artificial Sequence
<220>
   <223> Artificial
<400> 18
   gtctggctca cagaagtgag ctttgttttt tcc 33
<210> 19
   <211> 38
   <212> DNA
   <213> Artificial Sequence
<220>
   <223> Artificial
<400> 19
   cctcgagcag agcttcaatg acttcacccg ggtggtgg 38
<210> 20
   <211> 41
   <212> DNA
   <213> Artificial Sequence
<220>
   <223> Artificial
<400> 20
   gggatccatc ctccccgccc accacccggg tgaagtcatt g 41
<210> 21
   <211> 34
   <212> DNA
   <213> Artificial Sequence
<220>
   <223> Artificial
<400> 21
   ggaaaaaaca aagctcactc agagcttcaa tgac 34
<210> 22
   <211> 34
   <212> DNA
   <213> Artificial Sequence
<220>
   <223> Artificial
<400> 22
   gtcattgaag ctctgagtga gctttgtttt ttcc 34
<210> 23
   <211> 31
   <212> DNA
   <213> Artificial Sequence
<220>
   <223> Artificial
<400> 23
   gtgggatccg atgcacacaa gagtgaggtt g 31
<210> 24
   <211> 35
   <212> DNA
   <213> Artificial Sequence
<220>
   <223> Artificial
<400> 24
   cacggatccc tataagccta aggcagcttg acttg 35
<210> 25
   <211> 18
   <212> DNA
   <213> Artificial Sequence
<220>
   <223> Artificial
<400> 25
   caaggagacg ggcgctcc 18
<210> 26
   <211> 19
   <212> DNA
   <213> Artificial Sequence
<220>
   <223> Artificial
<400> 26
   gcccaaggag gggattggc 19
<210> 27
   <211> 30
   <212> DNA
   <213> Artificial Sequence
<220>
   <223> Artificial
<400> 27
   gtggaattca tggagctgag gccctggttg 30
<210> 28
   <211> 38
   <212> DNA
   <213> Artificial Sequence
<220>
   <223> Artificial
<400> 28
   cacgcggccg ctcactacag ccgttgcccc gcctccac 38
<210> 29
   <211> 704
   <212> PRT
   <213> Homo Sapiens
<400> 29
   Met Glu Leu Arg Pro Trp Leu Leu Trp Val Val Ala Ala Thr Gly Thr
   1               5                   10                  15
   Leu Val Leu Leu Ala Ala Asp Ala Gln Gly Gln Lys Val Phe Thr Asn
               20                  25                  30
   Thr Trp Ala Val Arg Ile Pro Gly Gly Pro Ala Val Ala Asn Ser Val
           35                  40                  45
   Ala Arg Lys His Gly Phe Leu Asn Leu Gly Gln Ile Phe Gly Asp Tyr
       50                  55                  60
   Tyr His Phe Trp His Arg Gly Val Thr Lys Arg Ser Leu Ser Pro His
   65                  70                  75                  80
   Arg Pro Arg His Ser Arg Leu Gln Arg Glu Pro Gln Val Gln Trp Leu
                   85                  90                  95
   Glu Gln Gln Val Ala Lys Arg Arg Thr Lys Arg Asp Val Tyr Gln Glu
               100                 105                 110
   Pro Thr Asp Pro Lys Phe Pro Gln Gln Trp Tyr Leu Ser Gly Val Thr
           115                 120                 125
   Gln Arg Asp Leu Asn Val Lys Ala Ala Trp Ala Gln Gly Tyr Thr Gly
       130                 135                 140
   His Gly Ile Val Val Ser Ile Leu Asp Asp Gly Ile Glu Lys Asn His
   145                 150                 155                 160
   Pro Asp Leu Ala Gly Asn Tyr Asp Pro Gly Ala Ser Phe Asp Val Asn
                   165                 170                 175
   Asp Gln Asp Pro Asp Pro Gln Pro Arg Tyr Thr Gln Met Asn Asp Asn
               180                 185                 190
   Arg His Gly Thr Arg Cys Ala Gly Glu Val Ala Ala Val Ala Asn Asn
           195                 200                 205
   Gly Val Cys Gly Val Gly Val Ala Tyr Asn Ala Arg Ile Gly Gly Val
       210                 215                 220
   Arg Met Leu Asp Gly Glu Val Thr Asp Ala Val Glu Ala Arg Ser Leu
   225                 230                 235                 240
   Gly Leu Asn Pro Asn His Ile His Ile Tyr Ser Ala Ser Trp Gly Pro
                   245                 250                 255
   Glu Asp Asp Gly Lys Thr Val Asp Gly Pro Ala Arg Leu Ala Glu Glu
               260                 265                 270
   Ala Phe Phe Arg Gly Val Ser Gln Gly Arg Gly Gly Leu Gly Ser Ile
           275                 280                 285
   Phe Val Trp Ala Ser Gly Asn Gly Gly Arg Glu His Asp Ser Cys Asn
       290                 295                 300
   Cys Asp Gly Tyr Thr Asn Ser Ile Tyr Thr Leu Ser Ile Ser Ser Ala
   305                 310                 315                 320
   Thr Gln Phe Gly Asn Val Pro Trp Tyr Ser Glu Ala Cys Ser Ser Thr
                   325                 330                 335
   Leu Ala Thr Thr Tyr Ser Ser Gly Asn Gln Asn Glu Lys Gln Ile Val
               340                 345                 350
   Thr Thr Asp Leu Arg Gln Lys Cys Thr Glu Ser His Thr Gly Thr Ser
           355                 360                 365
   Ala Ser Ala Pro Leu Ala Ala Gly Ile Ile Ala Leu Thr Leu Glu Ala
       370                 375                 380
   Asn Lys Asn Leu Thr Trp Arg Asp Met Gln His Leu Val Val Gln Thr
   385                 390                 395                 400
   Ser Lys Pro Ala His Leu Asn Ala Asn Asp Trp Ala Thr Asn Gly Val
                   405                 410                 415
   Gly Arg Lys Val Ser His Ser Tyr Gly Tyr Gly Leu Leu Asp Ala Gly
               420                 425                 430
   Ala Met Val Ala Leu Ala Gln Asn Trp Thr Thr Val Ala Pro Gln Arg
           435                 440                 445
   Lys Cys Ile Ile Asp Ile Leu Thr Glu Pro Lys Asp Ile Gly Lys Arg
       450                 455                 460
   Leu Glu Val Arg Lys Thr Val Thr Ala Cys Leu Gly Glu Pro Asn His
   465 470 475 480
   Ile Thr Arg Leu Glu His Ala Gln Ala Arg Leu Thr Leu Ser Tyr Asn
   485 490 495
   Arg Arg Gly Asp Leu Ala Ile His Leu Val Ser Pro Met Gly Thr Arg
   500 505 510
   Ser Thr Leu Leu Ala Ala Arg Pro His Asp Tyr Ser Ala Asp Gly Phe
   515 520 525
   Asn Asp Trp Ala Phe Thr Thr Thr His Ser Trp Asp Glu Asp Pro Ser
   530 535 540
   Gly Glu Trp Val Leu Glu Ile Glu Asn Thr Ser Glu Ala Asn Asn Tyr
   545 550 555 560
   Gly Thr Leu Thr Lys Phe Thr Leu Val Leu Tyr Gly Thr Ala Pro Glu
   565 570 575
   Gly Leu Pro Val Pro Pro Glu Ser Ser Gly Cys Lys Thr Leu Thr Ser
   580 585 590
   Ser Gln Ala Cys Val Val Cys Glu Glu Gly Phe Ser Leu His Gln Lys
   595 600 605
   Ser Cys Val Gln His Cys Pro Pro Gly Phe Ala Pro Gln Val Leu Asp
   610 615 620
   Thr His Tyr Ser Thr Glu Asn Asp Val Glu Thr Ile Arg Ala Ser Val
   625 630 635 640
   Cys Ala Pro Cys His Ala Ser Cys Ala Thr Cys Gln Gly Pro Ala Leu
   645 650 655
   Thr Asp Cys Leu Ser Cys Pro Ser His Ala Ser Leu Asp Pro Val Glu
   660 665 670
   Gln Thr Cys Ser Arg Gln Ser Gln Ser Ser Arg Glu Ser Pro Pro Gln
   675 680 685
   Gln Gln Pro Pro Arg Leu Pro Pro Glu Val Glu Ala Gly Gln Arg Leu
   690 695 700
<210> 30
   <211> 25
   <212> PRT
   <213> Artificial
<220>
   <223> Linker
<400> 30
   Ser Ser Gly Gly Ser Gly Gly Ser Gly Gly Ser Gly Gly Ser Gly Gly
   1               5                   10                  15
   Ser Gly Gly Ser Gly Gly Ser Gly Ser
               20                  25
<210> 31
   <211> 31
   <212> PRT
   <213> Artificial
<220>
   <223> Linker
<400> 31
   Ser Ser Asn Gly Ser Gly Gly Ser Gly Gly Ser Gly Gly Ser Asn Gly
   1               5                   10                  15
   Ser Gly Gly Ser Gly Gly Ser Gly Gly Ser Gly Gly Asn Gly Ser
               20                  25                  30
<210> 32
   <211> 17
   <212> PRT
   <213> Artificial
<220>
   <223> Linker
<400> 32
   Arg Ala Glu Thr Val Phe Pro Asp Val Asp Tyr Val Asn Ser Thr Glu
   1               5                   10                  15
   Ala
<210> 33
   <211> 14
   <212> PRT
   <213> Artificial
<220>
   <223> Linker
<400> 33
   Arg Ala Glu Thr Val Phe Pro Asp Val Asp Tyr Val Asn Ser
   1 5 10
<210> 34
   <211> 17
   <212> PRT
   <213> Artificial
<220>
   <223> Linker
<400> 34
   Arg Ala Glu Ala Val Phe Pro Asp Val Asp Tyr Val Asn Ser Thr Glu
   1 5 10 15
   Ala
<210> 35
   <211> 19
   <212> PRT
   <213> Artificial
<220>
   <223> Linker
<400> 35
   Arg Ala Glu Ala Val Phe Pro Asp Val Asp Tyr Val Asn Ser Thr Glu
   1 5 10 15
   Ala Gly Ser
<210> 36
   <211> 19
   <212> PRT
   <213> Artificial
<220>
   <223> Linker
<400> 36
   Ser Val Ser Gln Thr Ser Lys Leu Thr Arg Ala Glu Thr Val Phe Pro
   1 5 10 15
   Asp Val Asp
<210> 37
   <211> 21
   <212> PRT
   <213> Artificial
<220>
   <223> Linker
<400> 37
   Ser Val Ser Gln Thr Ser Lys Leu Thr Arg Ala Glu Thr Val Phe Pro
   1 5 10 15
   Asp Val Asp Gly Ser
   20
<210> 38
   <211> 19
   <212> PRT
   <213> Artificial
<220>
   <223> Linker
<400> 38
   Ser Val Ser Gln Thr Ser Lys Leu Thr Arg Ala Glu Thr Val Phe Pro
   1 5 10 15
   Asp Val Asp
<210> 39
   <211> 21
   <212> PRT
   <213> Artificial
<220>
   <223> Linker
<400> 39
   Ser Val Ser Gln Thr Ser Lys Leu Thr Arg Ala Glu Thr Val Phe Pro
   1 5 10 15
   Asp Val Asp Gly Ser
   20
<210> 40
   <211> 22
   <212> PRT
   <213> Artificial
<220>
   <223> Linker
<400> 40
   Ser Val Ser Gln Thr Ser Lys Leu Thr Arg Ala Glu Thr Val Phe Pro
   1 5 10 15
   Asp Val Asp Asn Gly Ser
   20
<210> 41
   <211> 19
   <212> PRT
   <213> Artificial
<220>
   <223> Linker
<400> 41
<210> 42
   <211> 19
   <212> PRT
   <213> Artificial
<220>
   <223> Linker
<400> 42
   Ser Val Ser Gln Thr Ser Lys Leu Thr Arg Thr Glu Thr Val Phe Pro
   1               5                   10                  15
   Asp Val Asp
<210> 43
   <211> 19
   <212> PRT
   <213> Artificial
<220>
   <223> Linker
<400> 43
   Ser Val Ser Gln Thr Ser Lys Leu Thr Arg Ser Glu Thr Val Phe Pro
   1               5                   10                  15
   Asp Val Asp
<210> 44
   <211> 19
   <212> PRT
   <213> Artificial
<220>
   <223> Linker
<400> 44
<210> 45
   <211> 19
   <212> PRT
   <213> Artificial
<220>
   <223> Linker
<400> 45
   Ser Val Ser Gln Thr Ser Lys Leu Thr Arg Thr Glu Ala Val Phe Pro
   1               5                   10                  15
   Asp Val Asp
<210> 46
   <211> 19
   <212> PRT
   <213> Artificial
<220>
   <223> Linker
<400> 46
   Ser Val Ser Gln Thr Ser Lys Leu Thr Arg Gly Glu Ala Val Phe Pro
   1               5                   10                  15
   Asp Val Asp
<210> 47
   <211> 14
   <212> PRT
   <213> Artificial
<220>
   <223> Linker
<400> 47
   Ser Lys Leu Thr Arg Ala Glu Thr Val Phe Pro Asp Val Asp
   1               5                   10
<210> 48
   <211> 14
   <212> PRT
   <213> Artificial
<220>
   <223> Linker
<400> 48
   Gln Ser Phe Asn Asp Phe Thr Arg Val Val Gly Gly Glu Asp
   1 5 10
<210> 49
   <211> 16
   <212> PRT
   <213> Artificial
<220>
   <223> Linker
<400> 49
   Gln Ser Phe Asn Asp Phe Thr Arg Val Val Gly Gly Glu Asp Gly Ser
   1 5 10 15
<210> 50
   <211> 14
   <212> PRT
   <213> Artificial
<220>
   <223> Linker
<400> 50
   Gln Ser Phe Asn Asp Phe Thr Arg Thr Val Gly Gly Glu Asp
   1 5 10
<210> 51
   <211> 14
   <212> PRT
   <213> Artificial
<220>
   <223> Linker
<400> 51
   Gln Ser Phe Asn Asp Phe Thr Arg Leu Val Gly Gly Glu Asp
   1 5 10
<210> 52
   <211> 14
   <212> PRT
   <213> Artificial
<220>
   <223> Linker
<400> 52
   Gln Ser Phe Asn Asp Phe Thr Arg Gly Val Gly Gly Glu Asp
   1 5 10
<210> 53
   <211> 17
   <212> PRT
   <213> Artificial
<220>
   <223> Linker
<400> 53
   Gln Ser Phe Asn Asp Phe Thr Arg Val Val Gly Gly Glu Asp Asn Gly
   1               5                   10                  15
   Ser
<210> 54
   <211> 15
   <212> PRT
   <213> Artificial
<220>
   <223> Linker
<400> 54
   Gln Ser Phe Asn Asp Phe Thr Arg Val Val Gly Gly Glu Asp Asn
   1               5                   10                  15
<210> 55
   <211> 7
   <212> PRT
   <213> Artificial
<220>
   <223> Linker
<400> 55
   Arg Ile Val Gly Gly Gln Glu
   1               5
<210> 56
   <211> 7
   <212> PRT
   <213> Artificial
<220>
   <223> Linker
<400> 56
   Arg Leu Val Gly Gly Gln Glu
   1               5
<210> 57
   <211> 7
   <212> PRT
   <213> Artificial
<220>
   <223> Linker
<400> 57
   Arg Thr Val Gly Gly Gln Glu
   1 5
<210> 58
   <211> 7
   <212> PRT
   <213> Artificial
<220>
   <223> Linker
<400> 58
   Arg Val Val Gly Gly Gln Glu
   1 5
<210> 59
   <211> 7
   <212> PRT
   <213> Artificial
<220>
   <223> Linker
<400> 59
   Arg Ala Val Gly Gly Gln Glu
   1 5
<210> 60
   <211> 7
   <212> PRT
   <213> Artificial
<220>
   <223> Linker
<400> 60
   Arg Gly Val Gly Gly Gln Glu
   1 5
<210> 61
   <211> 16
   <212> PRT
   <213> Artificial
<220>
   <223> Linker
<400> 61
   Pro Glu Arg Gly Asp Asn Asn Leu Thr Arg Ile Val Gly Gly Gln Glu
   1 5 10 15
<210> 62
   <211> 19
   <212> PRT
   <213> Artificial
<220>
   <223> Linker
<400> 62
   Ser Val Ser Gln Thr Ser Lys Leu Thr Arg Ala Glu Thr Val Phe Pro
   1 5 10 15
   Asp Val Asp
<210> 63
   <211> 15
   <212> PRT
   <213> Artificial
<220>
   <223> Linker
<400> 63
   Gln Ser Phe Asn Asp Phe Thr Arg Val Val Gly Gly Glu Asp Asn
   1 5 10 15
<210> 64
   <211> 19
   <212> PRT
   <213> Artificial
<220>
   <223> Linker
<400> 64
   Ser Val Ser Gln Thr Ser Lys Leu Thr Arg Ala Glu Thr Val Phe Pro
   1 5 10 15
   Asp Val Asp
<210> 65
   <211> 16
   <212> PRT
   <213> Artificial
<220>
   <223> Linker
<400> 65
   Lys Arg Asn Ala Ser Lys Pro Gln Gly Arg Ile Val Gly Gly Lys Val
   1 5 10 15
<210> 66
   <211> 16
   <212> PRT
   <213> Artificial
<220>
   <223> Linker
<400> 66
   Pro Glu Glu Pro Gln Leu Arg Met Lys Asn Asn Glu Glu Ala Glu Asp
   1 5 10 15
<210> 67
   <211> 19
   <212> PRT
   <213> Artificial
<220>
   <223> Linker
<400> 67
   Asp Asn Ser Pro Ser Phe Ile Gln Ile Arg Ser Val Ala Lys Lys His
   1 5 10 15
   Pro Lys Thr
<210> 68
   <211> 20
   <212> PRT
   <213> Artificial
<220>
   <223> Linker
<400> 68
   Leu Ser Lys Asn Asn Ala Ile Glu Pro Arg Ser Phe Ser Gln Asn Ser
   1 5 10 15
   Arg His Pro Ser
   20
<210> 69
   <211> 21
   <212> PRT
   <213> Artificial
<220>
   <223> Linker
<400> 69
   Asp Glu Asp Glu Asn Gln Ser Pro Arg Ser Phe Gln Lys Lys Thr Arg
   1 5 10 15
   His Tyr Phe Ile Ala
   20
<210> 70
   <211> 16
   <212> PRT
   <213> Artificial
<220>
   <223> Linker
<400> 70
   Ser Pro His Val Leu Arg Asn Arg Ala Gln Ser Gly Ser Val Pro Gln
   1 5 10 15
<210> 71
   <211> 24
   <212> PRT
   <213> Artificial
<220>
   <223> Linker
<400> 71
   Pro Glu Glu Pro Gln Leu Arg Met Lys Asn Asn Glu Glu Ala Glu Asp
   1 5 10 15
   Tyr Asp Asp Asp Leu Thr Asp Ser
   20
<210> 72
   <211> 31
   <212> PRT
   <213> Artificial
<220>
   <223> Linker
<400> 72
   Asp Asp Asp Asn Ser Pro Ser Phe Ile Gln Ile Arg Ser Val Ala Lys
   1 5 10 15
   Lys His Pro Lys Thr Trp Val His Tyr Ala Ala Glu Glu Glu Asp
   20 25 30
<210> 73
   <211> 28
   <212> PRT
   <213> Artificial
<220>
   <223> Linker
<400> 73
   Leu Ser Lys Asn Asn Ala Ile Glu Pro Arg Ser Phe Ser Gln Asn Ser
   1               5                   10                  15
   Arg His Pro Ser Thr Arg Gln Lys Gln Phe Asn Ala
               20                  25
<210> 74
   <211> 22
   <212> PRT
   <213> Artificial
<220>
   <223> Linker
<400> 74
   Asp Glu Asp Glu Asn Gln Ser Pro Arg Ser Phe Gln Lys Lys Thr Arg
   1               5                   10                  15
   His Tyr Phe Ile Ala Ala
               20
<210> 75
   <211> 32
   <212> PRT
   <213> Artificial
<220>
   <223> Linker
<400> 75
   Asp Tyr Gly Met Ser Ser Ser Pro His Val Leu Arg Asn Arg Ala Gln
   1               5                   10                  15
   Ser Gly Ser Val Pro Gln Phe Lys Lys Val Val Phe Gln Glu Phe Thr
               20                  25                  30
<210> 76
   <211> 24
   <212> PRT
   <213> Artificial
<220>
   <223> Linker
<400> 76
   Asp Ile Tyr Asp Glu Asp Glu Asn Gln Ser Pro Arg Ser Phe Gln Lys
   1 5 10 15
   Lys Thr Arg His Tyr Phe Ile Ala
   20
<210> 77
   <211> 17
   <212> PRT
   <213> Artificial
<220>
   <223> Linker
<400> 77
   Asp Asn Ser Pro Ser Phe Ile Gln Ile Arg Ser Val Ala Lys Lys His
   1 5 10 15
   Pro
<210> 78
   <211> 20
   <212> PRT
   <213> Artificial
<220>
   <223> Linker
<400> 78
   Leu Ser Lys Asn Asn Ala Ile Glu Pro Arg Ser Phe Ser Gln Asn Ser
   1 5 10 15
   Arg His Pro Ser
   20
<210> 79
   <211> 36
   <212> PRT
   <213> Artificial
<220>
   <223> Linker
<400> 79
   Ser Val Ser Gln Thr Ser Lys Leu Thr Arg Ala Glu Thr Val Phe Pro
   1 5 10 15
   Asp Val Thr Gln Pro Glu Arg Gly Asp Asn Asn Leu Thr Arg Ile Val
   20 25 30
   Gly Gly Gln Glu
   35
<210> 80
   <211> 23
   <212> PRT
   <213> Artificial
<220>
   <223> Linker
<400> 80
   Ser Lys Leu Thr Arg Ala Glu Thr Val Phe Pro Asp Asn Asn Leu Thr
   1               5                   10                  15
   Arg Ile Val Gly Gly Gln Glu
               20
<210> 81
   <211> 27
   <212> PRT
   <213> Artificial
<220>
   <223> Linker
<400> 81
   Arg Ala Glu Thr Val Phe Pro Asp Val Thr Gln Pro Glu Arg Gly Asp
   1               5                   10                  15
   Asn Asn Leu Thr Arg Ile Val Gly Gly Gln Glu
               20                  25
<210> 82
   <211> 22
   <212> PRT
   <213> Artificial
<220>
   <223> Linker
<400> 82
   Arg Ala Glu Thr Val Phe Pro Glu Arg Gly Asp Asn Asn Leu Thr Arg
   1               5                   10                  15
   Ile Val Gly Gly Gln Glu
               20
<210> 83
   <211> 32
   <212> PRT
   <213> Artificial
<220>
   <223> Linker
<400> 83
   Ser Val Ser Gln Thr Ser Lys Leu Thr Arg Ala Glu Thr Val Phe Pro
   1 5 10 15
   Asp Val Asp Tyr Val Asn Asn Leu Thr Arg Ile Val Gly Gly Gln Glu
   20 25 30
<210> 84
   <211> 30
   <212> PRT
   <213> Artificial
<220>
   <223> Linker
<400> 84
   Ser Val Ser Gln Thr Ser Lys Leu Thr Arg Ala Glu Thr Val Phe Pro
   1 5 10 15
   Asp Val Asp Asn Asn Leu Thr Arg Ile Val Gly Gly Gln Glu
   20 25 30
<210> 85
   <211> 30
   <212> PRT
   <213> Artificial
<220>
   <223> Linker
<400> 85
   Ser Val Ser Gln Thr Ser Lys Leu Thr Arg Ala Glu Thr Val Phe Pro
   1 5 10 15
   Asp Val Asp Asn Asn Leu Thr Arg Ile Val Gly Gly Gln Glu
   20 25 30
<210> 86
   <211> 52
   <212> PRT
   <213> Artificial
<220>
   <223> Linker
<400> 86
   Ser Val Ser Gln Thr Ser Lys Leu Thr Arg Ala Glu Thr Val Phe Pro
   1 5 10 15
   Asp Val Asp Tyr Val Asn Ser Thr Glu Ala Glu Thr Ile Leu Asp Asn
   20 25 30
   Ile Thr Gln Ser Thr Gln Ser Phe Asn Asp Phe Thr Arg Val Val Gly
   35 40 45
   Gly Glu Asp Ala
   50
<210> 87
   <211> 32
   <212> PRT
   <213> Artificial
<220>
   <223> Linker
<400> 87
   Ser Val Ser Gln Thr Ser Lys Leu Thr Arg Ala Glu Thr Val Phe Pro
   1 5 10 15
   Asp Val Gln Ser Phe Asn Asp Phe Thr Arg Val Val Gly Gly Glu Asp
   20 25 30
<210> 88
   <211> 32
   <212> PRT
   <213> Artificial
<220>
   <223> Linker
<400> 88
   Ser Val Ser Gln Thr Ser Lys Leu Thr Arg Ala Glu Thr Val Phe Pro
   1 5 10 15
   Asp Val Asp Ser Phe Asn Asp Phe Thr Arg Val Val Gly Gly Glu Asp
   20 25 30
<210> 89
   <211> 32
   <212> PRT
   <213> Artificial
<220>
   <223> Linker
<400> 89
   Ser Val Ser Gln Thr Ser Lys Leu Thr Arg Ala Glu Thr Val Phe Pro
   1 5 10 15
   Asp Val Asn Ala Ser Lys Pro Gln Gly Arg Ile Val Gly Gly Lys Val
   20 25 30
<210> 90
   <211> 32
   <212> PRT
   <213> Artificial
<220>
   <223> Linker
<400> 90
   Ser Val Ser Gln Thr Ser Lys Leu Thr Arg Ala Glu Thr Val Phe Pro
   1 5 10 15
   Asp Val Asn Ala Ser Lys Pro Gln Gly Arg Leu Val Gly Gly Lys Val
   20 25 30
<210> 91
   <211> 32
   <212> PRT
   <213> Artificial
<220>
   <223> Linker
<400> 91
   Ser Val Ser Gln Thr Ser Lys Leu Thr Arg Ala Glu Thr Val Phe Pro
   1 5 10 15
   Asp Val Asn Ala Ser Lys Pro Gln Gly Arg Thr Val Gly Gly Lys Val
   20 25 30
<210> 92
   <211> 19
   <212> PRT
   <213> Artificial
<220>
   <223> Linker
<400> 92
   Ser Val Ser Gln Thr Ser Lys Leu Thr Arg Ala Glu Thr Val Phe Pro
   1 5 10 15
   Asp Val Asp
<210> 93
   <211> 666
   <212> PRT
   <213> Homo Sapiens
<400> 93
   Tyr Asn Ser Gly Lys Leu Glu Glu Phe Val Gln Gly Asn Leu Glu Arg
   1               5                   10                  15
   Glu Cys Met Glu Glu Lys Cys Ser Phe Glu Glu Ala Arg Glu Val Phe
               20                  25                  30
   Glu Asn Thr Glu Arg Thr Thr Glu Phe Trp Lys Gln Tyr Val Asp Gly
           35                  40                  45
   Asp Gln Cys Glu Ser Asn Pro Cys Leu Asn Gly Gly Ser Cys Lys Asp
       50                  55                  60
   Asp Ile Asn Ser Tyr Glu Cys Trp Cys Pro Phe Gly Phe Glu Gly Lys
   65                  70                  75                  80
   Asn Cys Glu Leu Asp Val Thr Cys Asn Ile Lys Asn Gly Arg Cys Glu
                   85                  90                  95
   Gln Phe Cys Lys Asn Ser Ala Asp Asn Lys Val Val Cys Ser Cys Thr
               100                 105                 110
   Glu Gly Tyr Arg Leu Ala Glu Asn Gln Lys Ser Cys Glu Pro Ala Val
           115                 120                 125
   Pro Phe Pro Cys Gly Arg Val Ser Val Ser Gln Thr Ser Lys Leu Thr
       130                 135                 140
   Arg Ala Glu Thr Val Phe Pro Asp Val Asp Tyr Val Asn Ser Thr Glu
   145                 150                 155                 160
   Ala Glu Thr Ile Leu Asp Asn Ile Thr Gln Ser Thr Gln Ser Phe Asn
                   165                 170                 175
   Asp Phe Thr Arg Val Val Gly Gly Glu Asp Ala Lys Pro Gly Gln Phe
               180                 185                 190
   Pro Trp Gln Val Val Leu Asn Gly Lys Val Asp Ala Phe Cys Gly Gly
           195                 200                 205
   Ser Ile Val Asn Glu Lys Trp Ile Val Thr Ala Ala His Cys Val Glu
       210                 215                 220
   Thr Gly Val Lys Ile Thr Val Val Ala Gly Glu His Asn Ile Glu Glu
   225                 230                 235                 240
   Thr Glu His Thr Glu Gln Lys Arg Asn Val Ile Arg Ile Ile Pro His
   245 250 255
   His Asn Tyr Asn Ala Ala Ile Asn Lys Tyr Asn His Asp Ile Ala Leu
   260 265 270
   Leu Glu Leu Asp Glu Pro Leu Val Leu Asn Ser Tyr Val Thr Pro Ile
   275 280 285
   Cys Ile Ala Asp Lys Glu Tyr Thr Asn Ile Phe Leu Lys Phe Gly Ser
   290 295 300
   Gly Tyr Val Ser Gly Trp Gly Arg Val Phe His Lys Gly Arg Ser Ala
   305 310 315 320
   Leu Val Leu Gln Tyr Leu Arg Val Pro Leu Val Asp Arg Ala Thr Cys
   325 330 335
   Leu Arg Ser Thr Lys Phe Thr Ile Tyr Asn Asn Met Phe Cys Ala Gly
   340 345 350
   Phe His Glu Gly Gly Arg Asp Ser Cys Gln Gly Asp Ser Gly Gly Pro
   355 360 365
   His Val Thr Glu Val Glu Gly Thr Ser Phe Leu Thr Gly Ile Ile Ser
   370 375 380
   Trp Gly Glu Glu Cys Ala Met Lys Gly Lys Tyr Gly Ile Tyr Thr Lys
   385 390 395 400
   Val Ser Arg Tyr Val Asn Trp Ile Lys Glu Lys Thr Lys Leu Thr Ser
   405 410 415
   Val Ser Gln Thr Ser Lys Leu Thr Arg Ala Glu Thr Val Phe Pro Asp
   420 425 430
   Val Asp Glu Pro Lys Ser Cys Asp Lys Thr His Thr Cys Pro Pro Cys
   435 440 445
   Pro Ala Pro Glu Leu Leu Gly Gly Pro Ser Val Phe Leu Phe Pro Pro
   450 455 460
   Lys Pro Lys Asp Thr Leu Met Ile Ser Arg Thr Pro Glu Val Thr Cys
   465 470 475 480
   Val Val Val Asp Val Ser His Glu Asp Pro Glu Val Lys Phe Asn Trp
   485 490 495
   Tyr Val Asp Gly Val Glu Val His Asn Ala Lys Thr Lys Pro Arg Glu
               500                 505                 510
   Glu Gln Tyr Asn Ser Thr Tyr Arg Val Val Ser Val Leu Thr Val Leu
           515                 520                 525
   His Gln Asp Trp Leu Asn Gly Lys Glu Tyr Lys Cys Lys Val Ser Asn
       530                 535                 540
   Lys Ala Leu Pro Ala Pro Ile Glu Lys Thr Ile Ser Lys Ala Lys Gly
   545                 550                 555                 560
   Gln Pro Arg Glu Pro Gln Val Tyr Thr Leu Pro Pro Ser Arg Glu Glu
                   565                 570                 575
   Met Thr Lys Asn Gln Val Ser Leu Thr Cys Leu Val Lys Gly Phe Tyr
               580                 585                 590
   Pro Ser Asp Ile Ala Val Glu Trp Glu Ser Asn Gly Gln Pro Glu Asn
           595                 600                 605
   Asn Tyr Lys Thr Thr Pro Pro Val Leu Asp Ser Asp Gly Ser Phe Phe
       610                 615                 620
   Leu Tyr Ser Lys Leu Thr Val Asp Lys Ser Arg Trp Gln Gln Gly Asn
   625                 630                 635                 640
   Val Phe Ser Cys Ser Val Met His Glu Ala Leu His Asn His Tyr Thr
                   645                 650                 655
   Gln Lys Ser Leu Ser Leu Ser Pro Gly Lys
               660                 665
<210> 94
   <211> 7
   <212> PRT
   <213> Artificial
<220>
   <223> Linker
<400> 94
   Gly Gly Gly Gly Gly Gly Val
   1               5
<210> 95
   <211> 24
   <212> PRT
   <213> Artificial
<220>
   <223> Linker
<400> 95
   Ser Val Ser Gln Thr Ser Lys Leu Thr Arg Ala Glu Thr Val Phe Pro
   1               5                   10                  15
   Asp Val Asp Gly Ser Gly Gly Ser
               20
<210> 96
   <211> 18
   <212> PRT
   <213> Artificial
<220>
   <223> Linker
<400> 96
   Ser Val Ser Gln Thr Ser Lys Leu Thr Arg Ala Glu Thr Val Phe Pro
   1               5                   10                  15
   Asp Val
<210> 97
   <211> 15
   <212> PRT
   <213> Artificial
<220>
   <223> Linker
<400> 97
   Gln Thr Ser Lys Leu Thr Arg Ala Glu Thr Val Phe Pro Asp Val
   1               5                   10                  15
<210> 98
   <211> 13
   <212> PRT
   <213> Artificial
<220>
   <223> Linker
<400> 98
   Ser Lys Leu Thr Arg Ala Glu Thr Val Phe Pro Asp Val
   1               5                   10
<210> 99
   <211> 16
   <212> PRT
   <213> Artificial
<220>
   <223> Linker
<400> 99
   Ser Val Ser Gln Thr Ser Lys Leu Thr Arg Ala Glu Thr Val Phe Pro
   1 5 10 15
<210> 100
   <211> 15
   <212> PRT
   <213> Artificial
<220>
   <223> Linker
<400> 100
   Ser Val Ser Gln Thr Ser Lys Leu Thr Arg Ala Glu Thr Val Phe
   1 5 10 15
<210> 101
   <211> 12
   <212> PRT
   <213> Artificial
<220>
   <223> Linker
<400> 101
   Gln Thr Ser Lys Leu Thr Arg Ala Glu Thr Val Phe
   1 5 10
<210> 102
   <211> 10
   <212> PRT
   <213> Artificial
<220>
   <223> Linker
<400> 102
   Ser Lys Leu Thr Arg Ala Glu Thr Val Phe
   1 5 10
<210> 103
   <211> 13
   <212> PRT
   <213> Artificial
<220>
   <223> Linker
<400> 103
   Ser Val Ser Gln Thr Ser Lys Leu Thr Arg Ala Glu Thr
   1 5 10
<210> 104
   <211> 10
   <212> PRT
   <213> Artificial
<220>
   <223> Linker
<400> 104
   Gln Thr Ser Lys Leu Thr Arg Ala Glu Thr
   1 5 10
<210> 105
   <211> 8
   <212> PRT
   <213> Artificial
<220>
   <223> Linker
<400> 105
   Ser Lys Leu Thr Arg Ala Glu Thr
   1 5
<210> 106
   <211> 18
   <212> PRT
   <213> Artificial
<220>
   <223> Linker
<400> 106
   Gln Thr Ser Lys Leu Thr Arg Ala Glu Thr Val Phe Pro Asp Val Asp
   1 5 10 15
   Gly Ser
<210> 107
   <211> 16
   <212> PRT
   <213> Artificial
<220>
   <223> Linker
<400> 107
   Ser Lys Leu Thr Arg Ala Glu Thr Val Phe Pro Asp Val Asp Gly Ser
   1 5 10 15
<210> 108
   <211> 13
   <212> PRT
   <213> Artificial
<220>
   <223> Linker
<400> 108
   Gln Ser Phe Asn Asp Phe Thr Arg Val Val Gly Gly Glu
   1               5                   10
<210> 109
   <211> 18
   <212> PRT
   <213> Artificial
<220>
   <223> Linker
<400> 109
   Pro Glu Arg Gly Asp Asn Asn Leu Thr Arg Ile Val Gly Gly Gln Glu
   1               5                   10                  15
   Gly Ser
<210> 110
   <211> 15
   <212> PRT
   <213> Artificial
<220>
   <223> Linker
<400> 110
   Pro Glu Arg Gly Asp Asn Asn Leu Thr Arg Ile Val Gly Gly Gln
   1               5                   10                  15
<210> 111
   <211> 11
   <212> PRT
   <213> Artificial
<220>
   <223> Linker
<400> 111
   Asp Asn Asn Leu Thr Arg Ile Val Gly Gly Gln
   1               5                   10
<210> 112
   <211> 11
   <212> PRT
   <213> Artificial
<220>
   <223> Linker
<400> 112
   Ala Ser Lys Pro Gln Gly Arg Ile Val Gly Gly
   1               5                   10

## Description

The present invention relates to the field of modified therapeutic fusion proteins with increased half-life compared to their non-modified parent therapeutic polypeptides. The invention specifically relates to coagulation factors fused to half-life enhancing polypeptides (HLEPs), which are connected by linker peptides that are proteolytically cleavable for use in the treatment or prevention of a blood coagulation disorder. The cleavage of such linkers liberates the therapeutic polypeptide from any activity-compromising steric hindrance caused by the HLEP and thereby allows the generation of fusion proteins, which retain a high molar specific activity of the coagulation factor. In case the therapeutic fusion proteins are zymogens, those linkers are especially preferred that liberate the therapeutic polypeptide essentially simultaneous with its activation in vivo upon exposure to the corresponding protease(s). Another aspect of the present invention is a faster inactivation rate of a given coagulation factor once the coagulation factor is activated and the peptide linker is proteolytically cleaved in a coagulation-related mode and/or a faster elimination rate of a given coagulation factor once the coagulation factor is activated and the peptide linker is proteolytically cleaved in a coagulation-related mode compared to the corresponding fusion protein without cleavable linker.

The idea of the invention is demonstrated in particular by human vitamin K-dependent polypeptides Factor IX, Factor VII, and Factor VIIa but the concept also may be applied to other coagulation factors. Any half-life enhancing polypeptide (HLEP) may be connected to the therapeutic polypeptide by a cleavable linker peptide, but albumin or immunoglobulins or fragments derived thereof like the Fc fragment without an antigen binding domain are preferred HLEPs. The disclosure also relates to cDNA sequences coding for the therapeutic polypeptides and derivatives thereof genetically fused to a cDNA coding for HLEPs, such as human serum albumin linked by oligonucleotides that code for cleavable, intervening peptide linkers. Such encoded derivatives exhibit improved half-life and molar specific activities that are increased in comparison to their non-cleavable counterparts. The disclosure also relates to recombinant expression vectors containing such cDNA sequences, host cells transformed with such recombinant expression vectors, recombinant polypeptides and derivatives which do have biological activities comparable to the unmodified wild type therapeutic polypeptide but having improved half-lifes. The disclosure also relates to processes for the manufacture of such recombinant proteins and their derivatives. The disclosure also covers a transfer vector for use in human gene therapy, which comprises such modified DNA sequences useful to increase half-life in vivo.

### Background of the invention

Several recombinant, therapeutic polypeptides are commercially available for therapeutic and prophylactic use in humans. The patients in general benefit from the specific mode of action of the recombinant active ingredients but a disadvantage often is their limited availability due to their expensive and complex manufacturing processes. A reduction of the necessary dose or the frequency of administration of such products could improve this situation. A reduced frequency of administration could improve the convenience for the patient and, therefore, also the acceptance of the therapy. Several solutions have been described to achieve the goal of an increased in vivo half-life after administration. Solutions proposed recently include the formation of fusion proteins, especially in the case of polypeptides with a short in vivo half-life that can be increased significantly by fusion to a HLEP.

Ballance et al. (WO 01/79271) described fusion polypeptides of a multitude of different therapeutic polypeptides which, when fused to human serum albumin, are predicted to have an increased functional half-life in vivo and extended shelf-life. Long lists of potential fusion partners are described without showing by experimental data for almost any of these polypeptides that the respective albumin fusion proteins actually retain biological activity and have improved properties. Among the list of therapeutic polypeptides mentioned as Examples are Factor IX and FVII/FVIIa.. Also described are fusions of FIX and FVII/FVIIa in which there is a peptide linker between albumin and FIX or FVII/FVIIa. However, the use of cleavable linker peptides is not suggested.

Sheffield et al. (Sheffield W.P. et al. (2004), Br. J. Haematol. 126: 565-573) expressed a murine Factor IX albumin fusion protein composed of murine FIX, a linker of 8 amino acids (GPG₄TM), murine albumin and a peptide tag of 22 amino acids, and also a human Factor IX albumin fusion protein composed of human Factor IX, a linker of 7 amino acids (G₆V) and human albumin. Using a one-stage, FIX dependent clotting assay, the molar specific activities of the murine FIX-albumin fusion protein (MFUST) and the human FIX-albumin fusion protein (HFUS) were at least two- to three-fold lower than that of their unfused counterparts, an effect attributed at least partially to a slower proteolytic activation process by FXIa. Sheffield did not use or suggest using a cleavable linker between FIX and albumin.

Several patent applications describe the fusion of therapeutic polypeptides to immunoglobulin constant regions to extend the therapeutic polypeptide's in vivo half-life. WO 2002/04598, WO 2003/059935, WO 2004/081053, WO 2004/101740 and WO 2005/001025 include FIX as examples for the therapeutic polypeptide moiety. The latter two patent applications also describe FVII/FVIIa fused to immunoglobulin constant regions and find that fusion protein homodimers have inferior clotting activity compared to fusion proteins consisting of a monomer/dimer. Again, the use of cleavable linker peptides is not suggested.

In WO 91/09125 fusion proteins are disclosed that are joined by linkers which are cleavable by proteases of the blood coagulation cascade, but the fusion proteins are limited to those comprising fibrinolytic or antithrombotic proteins.
In WO 03/068934 chimeric molecules are described that are composed of at least one first component molecule, at least one linker and at least one second molecule, wherein the linker comprises an enzyme cleavage site to produce a non-naturally occurring linkage and cleavage site between the first and the second component molecule and wherein, upon cleavage of the chimeric molecule at the cleavage site, at least one of the component molecules is functionally active. The cleaving proteases may be coagulation factors like thrombin. Component molecules described among many others are FIX and FVIIa. However the therapeutic fusion proteins of the present invention are not disclosed, nor are improved properties of the therapeutic fusion proteins of the present invention disclosed such as increased molar specific activity, increased inactivation and/or elimination rates as compared to the therapeutic protein without cleavable linkers.
EP1444986 describes a pharmaceutical preparation for the treatment of blood-clotting disorders. WO96/01653 describes methods and compositions for the specific coagulation of vasculature. WO2004/101740 describes clotting factor-Fc chimeric proteins to treat hemophilia. EP1816201 describes a modified coagulation factor VIIa with extended half-life.

### Description of the invention

There is a great medical need for coagulation factors which have a long half-life. In the prior art fusions of coagulation factors to half-life enhancing polypeptides have been suggested to achieve this goal. However, once a coagulation factor is activated during coagulation either by proteolytic cleavage of the zymogen (like FIX) or by contact of an already proteolytically "pre"-activated factor to a second polypeptide (like FVIIa binding to Tissue Factor), it is no longer desirable to maintain the long half-life of the now activated coagulation factor as this might lead to thrombotic complications, as is already the case for a wild type coagulation factor as FVIIa (Aledort L.M., J Thromb Haemost 2(10): 1700-1708 (2004)) and should be even more relevant if the activated factor would have an increased half-life. It is therefore one objective of the present disclosure to provide long-lived coagulation factors, which after activation or after availability of a cofactor have a half-life comparable to that of an unmodified coagulation factor.

Fusions of the coagulation factors to half-life enhancing polypeptides as described in the prior art and as also shown in example 6 and 7 suffer in general from a reduced molar specific activity of the fused coagulation factor. Another aspect of the present disclosure is to provide coagulation factors with enhanced half-life, that show increased molar specific activity compared to the corresponding therapeutic fusion protein without a cleavable linker.

The invention is therefore about therapeutic fusion proteins for use in the treatment or prevention of a blood coagulation disorder comprising
a) a coagulation factor, wherein the coagulation factor is selected from the list consisting of Factor IX, Factor VIII, van Willebrand Factor, Factor V, Factor X, Factor XI, Factor XII, Factor XIII, Factor I, Factor II (Prothrombin), Protein C, Protein S, GAS6, and Protein Z ,
b) a half-life enhancing polypeptide , and
c) a peptide linker which joins the coagulation factor and the half-life enhancing polypeptide;
wherein the peptide linker is cleavable by proteases involved in coagulation or activated by coagulation enzymes and the therapeutic fusion protein has in comparison to the respective therapeutic fusion protein linked by a non-cleavable linker having the amino acid sequence GGGGGGV:
i) an increased molar specific activity in at least one coagulation-related assay; and
ii) an increased elimination rate of the activated coagulation factor after the peptide linker is proteolytically cleaved in a coagulation-related mode.

The invention also provides the therapeutic fusion proteins for use according to the invention formulated as a pharmaceutical composition.

As a consequence of the cleavable linker, after cleavage of the peptide linker in a coagulation-related mode, the coagulation factor more closely resembles the behaviour of the native, non-fused factor and does not show an increased half-life of the active factor with potentially prothrombotic effect.

Proteolytic cleavage in a coagulation-related mode in the sense of the invention, is any proteolytic cleavage that occurs as a consequence of the activation of at least one coagulation factor or coagulation cofactor.
The term "activated coagulation factor after the peptide linker is proteolytically cleaved in a coagulation-related mode" in the sense of the invention means that the coagulation factor is either activated almost in parallel to the proteolytic cleavage of the linker peptide, or that the coagulation factor was already activated before the proteolytic cleavage of the linker peptide. Activation may occur, for example by proteolytic cleavage of the coagulation factor or by binding to a cofactor.

The therapeutic fusion proteins can have an enhanced in vivo recovery as compared to the in vivo recovery of the unmodified coagulation factor.

Preferred are therapeutic fusion proteins which have an enhanced in vivo recovery compared to the unmodified coagulation factor by at least 10%, more preferred by at least 25% and most preferred by 40% or more.

Preferred HLEPs are albumin and fragments thereof and immunoglobulins including fragments thereof.

The linker region in a preferred embodiment comprises a sequence of the therapeutic polypeptide to be administered or a variant thereof, which should result in a decreased risk of neoantigenic properties (formation of a novel potentially immunogenic epitope due to the occurrence of a peptide within the therapeutic antigen which does not exist in human proteins) of the expressed fusion protein. Also in case the therapeutic protein is a zymogen (e.g. needs to be proteolytically activated) the kinetics of the peptide linker cleavage will more closely reflect the coagulation-related activation kinetics of the zymogen. Thus, in such preferred embodiments a zymogen and a corresponding linker are activated and respectively cleaved, with comparable kinetics. For this reason, the present invention also particularly relates to fusion proteins of a zymogen and a HLEP, where the kinetics of the linker cleavage by relevant proteases is not delayed by more than a factor of 3, and most preferably not by more than a factor of 2 compared to the kinetics of the zymogen activation.

In a further embodiment, the linker peptide comprises cleavage sites for more than one protease. This can be achieved either by a linker peptide that can be cleaved at the same position by different proteases or by a linker peptide that provides two or more different cleavage sites. This may be advantageous circumstances where the therapeutic fusion protein must be activated by proteolytic cleavage to achieve enzymatic activity and where different proteases may contribute to this activation step. This is the case, for example, upon activation of FIX, which can either be achieved by FXIa or by FVIIa/Tissue Factor (TF).

Preferred embodiments of the invention are therapeutic fusion proteins wherein the linker is cleavable by the protease, that activates the coagulation factor, thereby ensuring that the cleavage of the linker is linked to the activation of the coagulation factor at a site at which coagulation occurs.

Other preferred therapeutic fusion proteins according to the invention are those, wherein the linker is cleavable by the coagulation factor which is part of the therapeutic fusion protein once it is activated, thus also ensuring that cleavage of the fusion protein is connected with a coagulatory event.

Other preferred therapeutic fusion proteins according to the invention are those, wherein the linker is cleavable by a protease, which itself is activated directly or indirectly by the activity of the coagulation factor which is part of the therapeutic fusion protein, thus also ensuring that cleavage of the fusion protein is connected with a coagulatory event.

One class of most preferred therapeutic fusion proteins are those wherein the linker is cleavable by FXIa and/or by FVIIa/TF and the coagulation factor is FIX
The gist of the invention is demonstrated in particular by the vitamin K-dependent polypeptide Factor IX, cleavable linkers and albumin as the HLEP as well as its corresponding cDNA sequences. The disclosure also relates to cDNA sequences coding for any other coagulation factors which can be proteolytically activated or that are involved in the activation of other zymogens or polypeptides. These cDNAs are genetically fused to cDNA sequences coding for human serum albumin or other HLEPs, and are linked by oligonucleotides that code for intervening, cleavable peptide linkers. The expressed therapeutic fusion proteins exhibit molar specific activities which are increased in comparison to their non-cleavable counterparts. The disclosure also relates to recombinant expression vectors containing such fused cDNA sequences, host cells transformed with such recombinant expression vectors, recombinant therapeutic fusion proteins and derivatives that have biological activities almost comparable to the unmodified wild type therapeutic polypeptides but having improved in vivo half-life. The disclosure also relates to processes for the manufacture of such recombinant polypeptides and their derivatives. The disclosure also covers a transfer vector for use in human gene therapy, which comprises such modified DNA sequences useful to increase product levels in vivo.

Preferred therapeutic fusion proteins according to the invention are those that have a molar specific activity, in particular a molar specific activity in at least one coagulation-related assay that is at least 25% increased compared to that of the therapeutic fusion protein without a cleavable linker. More preferred are therapeutic fusion proteins in which the molar specific activity is increased by at least 50%, even more preferred those in which the molar specific activity is increased by at least 100%, in at least one of the different coagulation-related assays available.

Additional preferred embodiments of the present invention are therapeutic fusion proteins, wherein the inactivation rate of the activated coagulation factor after cleavage of the peptide linker which links the coagulation factor to the half-life enhancing polypeptide is increased by at least 10% as compared to the inactivation rate of the activated coagulation factor in a corresponding therapeutic fusion protein without a cleavable linker. More preferred are therapeutic fusion proteins in which the inactivation rate is increased by at least 25%, even more preferred those in which the inactivation rate is increased by at least 50%.

Additional preferred embodiments of the present invention are therapeutic fusion proteins, wherein the elimination rate of the coagulation factor after cleavage of the peptide linker that links the coagulation factor to the half-life enhancing polypeptide is increased by at least 10% as compared to the elimination rate of the coagulation factor in a corresponding therapeutic fusion protein without a cleavable linker. More preferred are therapeutic fusion proteins in which the elimination rate is increased by at least 25%, even more preferred those in which the elimination rate is increased by at least 50%.

### Detailed description

### Vitamin K-dependent polypeptides

Vitamin K-dependent polypeptides as one group of the therapeutic polypeptides are polypeptides that are γ-carboxylated enzymatically in the liver using vitamin K as a cofactor. Such vitamin K-dependent polypeptides e.g. are Factors II, VII, IX, X, Protein C, Protein S, GAS6, and Protein Z.

### Human FIX

Human FIX, one member of the group of vitamin K-dependent polypeptides, is a single-chain glycoprotein with a molecular weight of 57 kDa, which is secreted by liver cells into the blood stream as an inactive zymogen of 415 amino acids. It contains 12 γ-carboxy-glutamic acid residues localized in the N-terminal Gla-domain of the polypeptide. The Gla residues require vitamin K for their biosynthesis. Following the Gla domain there are two epidermal growth factor domains, an activation peptide, and a trypsin-type serine protease domain. Further posttranslational modifications of FIX encompass hydroxylation (Asp 64), N-(Asn157 and Asn167) as well as O-type glycosylation (Ser53, Ser61, Thr159, Thr169, and Thr172), sulfation (Tyr155), and phosphorylation (Ser158).

FIX is converted to its active form, Factor IXa, by proteolysis of the activation peptide at Arg145-Ala146 and Arg180-Val181 leading to the formation of two polypeptide chains, an N-terminal light chain (18 kDa) and a C-terminal heavy chain (28 kDa), which are held together by one disulfide bridge. Activation cleavage of Factor IX can be achieved in vitro e.g. by Factor XIa or Factor VIIa/TF. Factor IX is present in human plasma in a concentration of 5-10 µg/ml. Terminal plasma half-life of Factor IX in humans was found to be about 15 to 18 hours (White GC et al. 1997. Recombinant factor IX. Thromb Haemost. 78: 261-265; Ewenstein BM et al. 2002. Pharmacokinetic analysis of plasma-derived and recombinant F IX concentrates in previously treated patients with moderate or severe hemophilia B. Transfusion 42:190-197).

Hemophilia B is caused by non-functional or missing Factor IX and is treated with Factor IX concentrates from plasma or a recombinant form of Factor IX. As haemophilia B patients often receive at least biweekly prophylactic administrations of Factor IX to avoid spontaneous bleedings, it is desirable to increase the intervals of between administration by increasing the half-life of the Factor IX product applied. An improvement in plasma half-life would bring significant benefit to the patient. Up to now no pharmaceutical preparation of a Factor IX with improved plasma half-life is commercially available nor have any data been published showing F IX variants with prolonged in vivo half-life and almost unchanged molar specific activity in coagulation-related assays. Therefore, a great medical need still exists to develop forms of Factor IX which have a longer functional half-life in vivo.

### Factor VII and Factor Vila

FVII is a single-chain glycoprotein with a molecular weight of 50 kDa, which is secreted by liver cells into the blood stream as an inactive zymogen of 406 amino acids. FVII is converted to its active form Factor VIIa, by proteolysis of the single peptide bond at Arg152-Ile153 leading to the formation of two polypeptide chains, a N-terminal light chain (24 kDa) and a C-terminal heavy chain (28 kDa), which are held together by one disulfide bridge. In contrast to other vitamin K-dependent coagulation factors, no activation peptide is cleaved off during activation. Activation cleavage of Factor VII can be achieved in vitro, for example, by Factor Xa, Factor IXa, Factor VIIa, Factor XIIa, Factor Seven Activating Protease (FSAP), and thrombin. Mollerup et al. (Biotechnol. Bioeng. (1995) 48: 501-505) reported that some cleavage also occurs in the heavy chain at Arg290 and/or Arg315.

Factor VII is present in plasma in a concentration of 500 ng/ml. About 1% or 5 ng/ml of Factor VII is present as activated Factor VIIa. The terminal plasma half-life of Factor VII was found to be about 4 hours and that of Factor VIIa about 2 hours.

By administering supraphysiological concentrations of Factor VIIa hemostasis can be achieved bypassing the need for Factor Villa and Factor IXa. The cloning of the cDNA for Factor VII (US 4,784,950) made it possible to develop activated Factor VII as a pharmaceutical. Factor VIIa was successfully administered for the first time in 1988. Ever since the number of indications of Factor VIIa has grown steadily showing a potential to become an universal hemostatic agent to stop bleeding (Erhardtsen, 2002). However, the short terminal half-life of Factor VIIa of approximately 2 hours and reduced in vivo recovery is limiting its application. Therefore, a great medical need still exists to develop forms of Factor VIIa which have an improved half-life but otherwise almost uncompromised molar specific activity, inactivation kinetics, and/or elimination kinetics after start of coagulation.

### Therapeutic fusion proteins

"Therapeutic fusion proteins" in the sense of this invention are coagulation factors selected from the list consisting of Factor IX, Factor VIII, van Willebrand Factor, Factor V, Factor X, Factor XI, Factor XII, Factor XIII, Factor I, Factor II (Prothrombin), Protein C, Protein S, GAS6, and Protein Z fused to a half-life enhancing polypeptide that upon administration to a human or animal can produce a prophylactic or therapeutic effect. These therapeutic fusion proteins may be administered to a human or an animal via intravenous, intramuscular, oral, topical, parenteral or other routes. Specific classes of therapeutic fusion proteins covered, i.e. by the examples in this invention, are coagulation factors like e.g. vitamin K-dependent polypeptides linked to half-life enhancing polypeptides like e.g. albumin and immunoglobulins and their fragments or derivatives. The expression "therapeutic fusion protein" is used interchangeable with "fusion protein".

### Half-life enhancing polypeptide (HLEP)

Albumin, albumin family members and immunoglobulines and their fragments or derivatives have been described above as examples of half-life enhancing polypeptides (HLEPs). The terms "human serum albumin" (HSA) and "human albumin" (HA) are used interchangeably in this application. The terms "albumin" and "serum albumin" are broader, and encompass human serum albumin (and fragments and variants thereof) as well as albumin from other species (and fragments and variants thereof).

As used herein, "albumin" refers collectively to albumin polypeptide or amino acid sequence, or an albumin fragment or variant having one or more functional activities (e.g., biological activities) of albumin. In particular, "albumin" refers to human albumin or fragments thereof, especially the mature form of human albumin as shown in SEQ ID No:1 herein or albumin from other vertebrates or fragments thereof, or analogs or variants of these molecules or fragments thereof.

The albumin portion of the albumin fusion proteins may comprise the full length of the HA sequence as described above, or may include one or more fragments thereof that are capable of stabilizing or prolonging the therapeutic activity. Such fragments may be of 10 or more amino acids in length or may include about 15, 20, 25, 30, 50, or more contiguous amino acids from the HA sequence or may include part or all of specific domains of HA.

The albumin portion of the albumin fusion proteins of the disclosure may be a variant of normal HA, either natural or artificial. The therapeutic polypeptide portion of the fusion proteins of the invention may also be variants of the corresponding therapeutic polypeptides as described herein. The term "variants" includes insertions, deletions, and substitutions, either conservative or non-conservative, either natural or artificial, where such changes do not substantially alter the active site, or active domain that confers the therapeutic activities of the therapeutic polypeptides.

In particular, the albumin fusion proteins of the disclosure may include naturally occurring polymorphic variants of human albumin and fragments of human albumin. The albumin may be derived from any vertebrate, especially any mammal, for example human, cow, sheep, or pig. Non-mammalian albumins include, but are not limited to, hen and salmon. The albumin portion of the albumin-linked polypeptide may be from a different animal than the therapeutic polypeptide portion.

Generally speaking, an albumin fragment or variant will be at least 10, preferably at least 40, most preferably more than 70 amino acids long. The albumin variant may preferentially consist of or alternatively comprise at least one whole domain of albumin or fragments of said domains, for example domains 1 (amino acids 1-194 of SEQ ID NO:1), 2 (amino acids 195-387 of SEQ ID NO: 1), 3 (amino acids 388-585 of SEQ ID NO: 1), 1 + 2 (1-387 of SEQ ID NO: 1), 2 + 3 (195-585 of SEQ ID NO: 1) or 1 + 3 (amino acids 1-194 of SEQ ID NO: 1 + amino acids 388-585 of SEQ ID NO: 1). Each domain is itself made up of two homologous subdomains namely 1-105, 120-194, 195-291, 316-387, 388-491 and 512-585, with flexible inter-subdomain linker regions comprising residues Lys106 to Glu119, Glu292 to Val315, and Glu492 to Ala511.

The albumin portion of an albumin fusion protein of the disclosure may comprise at least one subdomain or domain of HA or conservative modifications thereof.

All fragments and variants of albumin are encompassed by the disclosure as fusion partners of a coagulation factor as long as they lead to a half-life extension of the therapeutic fusion protein in plasma of at least 25% as compared to the non-fused coagulation factor.

Besides albumin, alpha-fetoprotein, another member of the albumin family, has been claimed to enhance the half-life of an attached therapeutic polypeptide in vivo (WO 2005/024044). The albumin family of proteins, evolutionarily related serum transport proteins, consists of albumin, alpha-fetoprotein (AFP; Beattie & Dugaiczyk 1982. Gene 20:415-422), afamin (AFM; Lichenstein et al. 1994. J. Biol. Chem. 269:18149-18154) and vitamin D binding protein (DBP; Cooke & David 1985. J. Clin. Invest. 76:2420-2424). Their genes represent a multigene cluster with structural and functional similarities mapping to the same chromosomal region in humans, mice and rat. The structural similarity of the albumin family members suggest their usability as HLEPs. Also disclosed herein is therefore the use of such albumin family members, fragments and variants thereof as HLEPs. The term "variants" includes insertions, deletions and substitutions, either conservative or non-conservative as long as the desired function is still present.

Albumin family members may comprise the full length of the respective protein AFP, AFM and DBP, or may include one or more fragments thereof that are capable of stabilizing or prolonging the therapeutic activity. Such fragments may be of 10 or more amino acids in length or may include about 15, 20, 25, 30, 50, or more contiguous amino acids of the respective protein sequence or may include part or all of specific domains of the respective protein, as long as the HLEP fragments provide a half-life extension of at least 25% as compared to the non-fused coagulation factor. Albumin family members of the therapeutic fusion proteins of the disclosure may include naturally occurring polymorphic variants of AFP, AFM and DBP.

IgG and IgG-fragments may also be used as HLEPs, as long as the HLEP fragments provide a half-life extension of at least 25% as compared to the non-fused coagulation factor. The therapeutic polypeptide portion is connected to the IgG or the IgG fragments via a cleavable linker that allows high molar specific activities of the fusion protein. Examples for factor VII/VIIa and factor IX IgG fusion molecules are found, e.g., in WO 2005/001025. It discloses i.e. a homodimer comprised of two factor VII (factor VIIa) molecules and two Fc molecules and a monomer/dimer hybrid comprised of one FVII (FVIIa) molecule and two Fc molecules, the monomer/dimer showing an about four times greater clotting activity than the homodimer. A linker sequence of the present invention liberating the FVII (FVIIa) molecules upon cleavage by a protease of the coagulation cascade like, e.g., FXIa, FXa, or FIXa could be able to elevate the clotting activity of the constructs and especially that of the homodimer to an activity level comparable to the monomer/dimer or even higher. A FIX-Fc fusion protein with cleavable linker is exemplarily shown in SEQ ID No 93. Cleavable linkers such as those shown in table 3a and 3b may be applied in this case.

The invention specifically relates to fusion proteins comprising linking a coagulation factor to the N- or C-terminus of a HLEP or fragment or variant thereof such that an intervening cleavable peptide linker is introduced between the therapeutic polypeptide and the HLEP such that the fusion protein formed has an increased in vivo half-life compared to the coagulation factor which has not been linked to a HLEP and that the fusion protein has an at least 25% higher molar specific activity compared to the corresponding fusion protein with non-cleavable linker in at least one of the different coagulation-related assays available.

"Coagulation factor" as used in this application include, but is not limited to, polypeptides consisting of Factor IX, Factor VII, Factor VIII, von Willebrand Factor, Factor V, Factor X, Factor XI, Factor XII, Factor XIII, Factor I, Factor II (Prothrombin), Protein C, Protein S, GAS6, or Protein Z as well as their activated forms. Furthermore, useful therapeutic polypeptides may be wild-type polypeptides or may contain mutations. Degree and location of glycosylation or other post-translation modifications may vary depending on the chosen host cells and the nature of the host cellular environment. When referring to specific amino acid sequences, posttranslational modifications of such sequences are encompassed in this application.

"Coagulation factor" within the above definition includes polypeptides that have the natural amino acid sequence including any natural polymorphisms. It also includes polypeptides with a slightly modified amino acid sequence, for instance, a modified N-terminal or C-terminal end including terminal amino acid deletions or additions, as long as those polypeptides substantially retain the activity of the respective therapeutic polypeptide. Variants included differ in one or more amino acid residues from the wild type sequence. Examples of such differences may include truncation of the N- and/or C-terminus by one or more amino acid residues (e.g. preferably 1 to 30 amino acid residues), or addition of one or more extra residues at the N-and/or C-terminus, as well as conservative amino acid substitutions, i.e. substitutions performed within groups of amino acids with similar characteristics, e.g. (1) small amino acids, (2) acidic amino acids, (3) polar amino acids, (4) basic amino acids, (5) hydrophobic amino acids, and (6) aromatic amino acids. Examples of such conservative substitutions are shown in the following table.

**Table 1**

| | | | | |
|---|---|---|---|---|
| (1) | Alanine | Glycine | | |
| (2) | Aspartic acid | Glutamic acid | | |
| (3a) | Asparagine | Glutamine | | |
| (3b) | Serine | Threonine | | |
| (4) | Arginine | Histidine | Lysine | |
| (5) | Isoleucine | Leucine | Methionine | Valine |
| (6) | Phenylalanine | Tyrosine | Tryptophane | |

The in vivo half-life of the fusion proteins of the invention, in general determined as terminal half-life or β-half-life, is usually at least about 25%, preferable at least about 50%, and more preferably more than 100% higher than the in vivo half-life of the non-fused polypeptide.

The fusion proteins of the present invention have at least a 25%, preferably at least a 50%, more preferably an at least 100% increased molar specific activity compared to the corresponding fusion proteins without cleavable linkers.

The molar specific activity (or molar specific coagulation-related activity as considered here in particular) in this regard is defined as the activity expressed per mole (or e.g. nmole) of the therapeutic polypeptide or therapeutic fusion protein of interest. Calculation of the molar specific activity allows a direct comparison of the activity of the different constructs which is not affected by the different molecular weights or optical densities of the polypeptides studied. The molar specific activity may be calculated as exemplified in table 2 below for FIX and a FIX-HSA fusion protein.

**Table 2: Calculation of molar specific activity as shown for a purified FIX-HSA fusion protein**

| Product | OD_{(280nm, 1%)} | MW | Activity/Vol/OD₂₈₀ (IU/L/OD₂₈₀) | Molar optical density (OD₍₂₈₀₎ at 1 mol/L) | Calculation of molar specific activity (IU/mol) |
|---|---|---|---|---|---|
| FIX | 13.3 ¹⁾ | 57 000 | determined for product | 75810 (= MW x OD_{(280, 1%)}/10) | =(Activity/Vol/OD₂₈₀) x (OD₂₈₀ at 1 mol/L) |
| HSA | 5.7 ²⁾ | 66 300 | | 37791 (= MW x OD_{(280, 1%)}/10) | |
| FIX-HSA | | | determined for product | 113601 (= sum of molar optical density of FIX and HSA) | = (Activity/Vol/OD₂₈₀) x (OD₂₈₀ at 1 mol/L) |

| | | | | | |
|---|---|---|---|---|---|
| ¹⁾ R.D. Di Scipio et al., Biochem. 16: 698-706 (1977) ²⁾ C. Chaudhury et al, J. Exp. Med. 197(3): 315-322 (2003) | | | | | |

In order to determine a molar specific coagulation-related activity, any assay may be used that determines enzymatic or cofactor activities that are relevant to the coagulation process.

Therefore "coagulation-related assays" in the sense of the invention is any assay which determines enzymatic or cofactor activities that are of relevance in the coagulation process or that is able to determine that either the intrinsic or the extrinsic coagulation cascade has been activated. The "coagulation-related" assay thus may be direct coagulation assays like aPTT, PT, or the thrombin generation assays. However, other assays like, e.g., chromogenic assays applied for specific coagulation factors are also included. Examples for such assays or corresponding reagents are Pathromtin® SL (aPTT assay, Dade Behring) or Thromborel® S (Prothrombin time assay, Dade Behring) with corresponding coagulation factor deficient plasma (Dade Behring), Thrombin generation assay kits (Technoclone, Thrombinoscope) using e.g. coagulation factor deficient plasma, chromogenic assays like Biophen Factor IX (Hyphen BioMed), Staclot® FVIIa-rTF (Roche Diagnostics GmbH), Coatest® Factor VIII:C/4 (Chromogenix), or others.

For purposes of this invention, an increase in any one of the above assays or an equivalent coagulation-related assay is considered to show an increase in molar specific activity. For example, a 25% increase refers to a 25% increase in any of the above or an equivalent assay.

To determine whether therapeutic fusion proteins fall within the scope of the present invention, the standard against which the molar specific activity of these proteins is compared is a construct in which the respective coagulation factor and the respective HLEP are linked by a non-cleavable linker having the amino acid sequence GGGGGGV.

In the case of FIX, aPTT assays are often used for determination of coagulation activity. Such a coagulation assay (aPTT assay) is described in example 5 in more detail. However, other coagulation-related assays or assay principles may be applied to determine molar specific activity for FIX.

Recombinant therapeutic polypeptide drugs are usually expensive and not all countries can afford costly therapies based on such drugs. Increasing the in vivo recovery of such drugs could make the use of these products cheaper and subsequently more patients would benefit from them. In the case of the fusion proteins of the present invention an increased in vivo recovery would also be a desirable advantage. "In vivo recovery" in the sense of the invention means the amount of product found in blood or plasma shortly after administration of the product. Therefore, for detection of the in vivo recovery in general the plasma content is determined a few minutes (e.g. 5 or 15 min) after administration of the product.

Although it is desirable to have a high in vivo recovery and a long half-life for a non-activated coagulation factor, it is advantageous to limit the half-life of a coagulation factor after its activation or the activation of its co-factor in order to avoid a prothrombotic risk. Therefore, after the coagulation process has been initiated, the half-life of the active coagulation factor should again be reduced. This can either be achieved by enhancing inactivation in a coagulation-related mode or by elimination of the coagulation factor.

Inactivation according to the present invention means the decrease of activity of the therapeutic polypeptide which can be caused, for example, by a complex formation of a coagulation factor and an inhibitor of the corresponding coagulation factor or by further proteolytic cleavage as known, e.g., in the case of FVIII and FV.

The inactivation rate of an activated therapeutic fusion protein is defined as the rate the activity is declining, e.g., by reaction with inhibitors or by proteolytic inactivation. The inactivation rate may be measured by following the molar specific activity of the activated coagulation factor over time in the presence of physiologic amounts of inhibitors of this coagulation factor. Alternatively, the inactivation rate may be determined after administration of the activated product to an animal followed by testing of plasma samples at an appropriate time frame using activity and antigen assays.

When for therapeutic fusion proteins a determination is needed whether these proteins fall within the scope of the present invention, the standard against which the inactivation rate of these therapeutic proteins is compared to, is a construct in which the respective coagulation factor and the respective HLEP are joined by a non-cleavable linker having the amino acid sequence GGGGGGV.

The elimination rate of an activated therapeutic fusion protein is defined as the rate the polypeptide is eliminated from the circulation of humans or animals. The elimination rate may be determined by measuring the pharmacokinetics of the activated, therapeutic fusion protein after intravenous administration. Using an antigen assay, the elimination by direct removal from the circulation can be determined. Using an activity assay in addition, a specific removal and inactivation rate may be determined.

When for therapeutic fusion proteins a determination is needed whether these proteins fall within the scope of the present invention, the standard against which the elimination rate of these proteins is compared to, is a construct in which the respective coagulation factor and the respective HLEP are joined by the non-cleavable linker having the amino acid sequence GGGGGGV.

According to this invention, the therapeutic polypeptide moiety is coupled to the HLEP moiety by a cleavable peptide linker. The linker should be non-immunogenic and should be flexible enough to allow cleavage by proteases. The cleavage of the linker should proceed comparably fast as the activation of the therapeutic polypeptide within the fusion protein, if the fusion protein is a zymogen.

The cleavable linker preferably comprises a sequence derived from
a) the therapeutic polypeptide to be administered itself if it contains proteolytic cleavage sites that are proteolytically cleaved during activation of the therapeutic polypeptide,
b) a substrate polypeptide of this therapeutic polypeptide, or
c) a substrate polypeptide cleaved by a protease which is activated or formed by the direct or indirect involvement of the therapeutic polypeptide.

The linker region in a more preferred embodiment comprises a sequence of the therapeutic polypeptide to be applied, which should result in a decreased risk of neoantigenic properties of the expressed fusion protein. Also in case the therapeutic protein is a zymogen (e.g. needs to be proteolytically activated) the kinetics of the peptide linker cleavage will more closely reflect the coagulation-related activation kinetics of the zymogen.

In a preferred embodiment, the therapeutic polypeptide is FIX zymogen and the HLEP is albumin. In this case the linker sequence is either derived from the sequences of the activation regions of FIX, from the cleavage region of any substrate of FIX like FX or FVII or from the cleavage region of any substrate polypeptide that is cleaved by a protease in whose activation FIXa is involved.

In a highly preferred embodiment the linker peptide is derived from FIX itself. In another preferred embodiment the linker peptide is derived from FX or FVII. In another preferred embodiment the linker sequence comprises two cleavage sequences that can be cleaved by FXIa or FVIIa/TF, two physiologically relevant activators of FIX.

Exemplary combinations of therapeutic polypeptide, cleavable linker and HLEP with coagulation factors selected from the list consisting of Factor IX, Factor VIII, van Willebrand Factor, Factor V, Factor X, Factor XI, Factor XII, Factor XIII, Factor I, Factor II (Prothrombin), Protein C, Protein S, GAS6, and Protein Z are included in the constructs listed in tables 3a and 3b but are not limited to these:

**Table 3a: Examples of possible constructs**

| **Coagulation factor** | **Linker** | **HLEP** | **Linker derived from (with modification, if applicable)** | **SEQ ID NO:** |
|---|---|---|---|---|
| | **Linker not cleavable or notsufficiently rapidly cleavable** | | | |
| FIX | - | HSA | | |
| FIX | RI | HSA | | |
| FIX | GGGGGGV(Sheffield et al.) | HSA | | 94 |
| FIX | (GGS)nGS | HSA | | |
| FIX | SS(GGS)₇ GS | HSA | | 30 |
| FIX | SSNGS(GGS)₃NGS(GGS)₃GGNGS | HSA | | 31 |
| | | | | |

| | **Linker with one cleavage site** | | | |
|---|---|---|---|---|
| FIX (1-412) | SVSQTSKLT**R**AETVFPDVD | HSA | FIX | 36 |
| FIX (1-412) | SVSQTSKLT**R**AETVFPDVD GS | HSA | FIX | 37 |
| FIX | SVSQTSKLT**R**AETVFPDVD | HSA | FIX | 38 |
| FIX | SVSQTSKLT**R**AETVFPDVD GS GGS | HSA | FIX | 95 |
| FIX | SVSQTSKLT**R**AETVFPDVD GS | HSA | FIX | 39 |
| FIX | SVSQTSKLT**R**AETVFPDVD NGS | HSA | FIX | 40 |
| FIX | SVSQTSKLT**R**AETVFPDV | HSA | FIX | 96 |
| FIX | QTSKLT**R**AERVFPDV | HSA | FIX | 97 |
| FIX | SKLT**R**AETVFPDV | HSA | FIX | 98 |
| FIX | SVSQTSKLT**R** AETVFP | HSA | FIX | 99 |
| FIX | SVSQTSKLT**R**AETVF | HSA | FIX | 100 |
| FIX | QTSKLT**R**AETVF | HSA | FIX | 101 |
| FIX | SKLT**R**AETVF | HSA | FIX | 102 |
| FIX | SVSQTSKLT**R**AET | HSA | FIX | 103 |
| FIX | QTSKLT**R** AET | HSA | FIX | 104 |
| FIX | SKLT**R**AET | HSA | FIX | 105 |
| FIX | SVSQTSKLT**R**GETVFPDVD | HSA | FIX | 41 |
| FIX | SVSQTSKLT**R**TETVFPDVD | HSA | FIX | 42 |
| FIX | SVSQTSKLT**R**SETVFPDVD | HSA | FIX | 43 |
| FIX | SVSQTSKLT**R** LETVFPDVD | HSA | FIX | 44 |
| FIX | SVSQTSKLT**R**TEAVFPDVD | HSA | FIX | 45 |
| FIX | SVSQTSKLT**R**GEAVFPDVD | HSA | FIX | 46 |
| FIX | QTSKLT**R**AETVFPDVD GS | HSA | FIX | 106 |
| FIX | SKLT**R**AETVFPDVD GS | HSA | FIX | 107 |
| FIX | SKLT**R**AETVFPDVD | HSA | FIX | 47 |
| FIX | QSFNDFT**R**VVGGED | HSA | FIX | 48 |
| FIX | QSFNDFT**R**VVGGED GS | HSA | FIX | 49 |
| FIX | QSFNDFT**R** VVGGE | HSA | FIX | 108 |
| FIX | QSFNDFT**R** TVGGED | HSA | FIX | 50 |
| FIX | QSFNDFT**R** LVGGED | HSA | FIX | 51 |
| FIX | QSFNDFT**R**GVGGED | HSA | FIX | 52 |
| FIX | QSFNDFT**R**VVGGED NGS | HSA | FIX | 53 |
| FIX | QSFNDFT**R**VVGGEDN | HSA | FIX | 54 |
| FIX | PERGDNNLT**R**IVGGQE GS | HSA | FX | 109 |
| FIX | PERGDNNLT**R**IVGGQE | HSA | FX | 61 |
| FIX | PERGDNNLT**R** IVGGQ | HSA | FX | 110 |
| FIX | DNNLT**R** IVGGQ | HSA | FX | 111 |
| FIX | SVSQTSKLT**R** AETVFPDVD | Fc | FIX | 62 |
| FIX | QSFNDFT**R** VVGGED N | Fc | FIX | 63 |
| FIX (1-412) | SVSQTSKLT**R** AETVFPDVD | Fc | FIX | 64 |
| FIX | ASKPQG**R** IVGG | HSA de IDAH | FVII | 112 |
| FIX | KRNASKPQG**R** IVGGKV | HSA | FVII | 65 |
| FIX | PEEPQL**R** MKNNEEAED | HSA | FVIII | 66 |
| FIX | DNSPSFIQI**R**SVAKKHPKT | HSA | FVIII | 67 |
| FIX | LSKNNAIEP**R**SFSQNSRHPS | HSA | FVIII | 68 |
| FIX | DEDENQSP**R**SFQKKTRHYFIA | HSA | FVIII | 69 |
| FIX | SPHVLRN**R**AQSGSVPQ | HSA | FVIII | 70 |
| FVII or FVI**I**a | PEEPQL**R** MKNNEEAEDYDDDLTDS | HSA | FVIII | 71 |
| FVII or FVIIa | DDDNSPSFIQI**R** SVAKKHPKTWVHYAAEEED | HSA | FVIII | 72 |
| FVII or FVIIa | LSKNNAIEP**R** SFSQN SRHPSTR QKQFNA | HSA | FVIII | 73 |
| FVI**I** or FVIIa | DEDEN QSP**R** SFQKKTRH YFIAA | HSA | FVIII | 74 |
| FVI**I** or FVIIa | DYGMSSSPHVLRN**R** AQSGSVPQFKKVVFQEFT | HSA | FVIII | 75 |
| FVIII | Derived from cleavage sites of FVIII, FIX, or Fibrinogen | HSA | FVIII, FIX or Fgn | |
| VWF | Derived from cleavage sites of VWF, FVIII, or FIX | HSA | FIX, FVIII, VWF | |
| VWF | DIYDEDENQSP**R** SFQKKTRHYFIA | HSA | FVIII | 76 |
| VWF | DNSPSFIQI**R** SVAKKHP | HSA | FVIII | 77 |
| VWF | LSKNNAIEP**R** SFSQNSR HPS | HSA | FVIII | 78 |

In the case of linkers derived from the N-terminal region of the FIX activation peptide, according to the natural polymorphism T148-A148 the sequences may also contain A instead of T at this position.

**Table 3b: Examples of possible constructs with two or more cleavage sites**

| **Coagulation factor** | **Linker** | **HLEP** | **Linker derived from (partially incl. Modifications)** | **SEQ ID NO:** |
|---|---|---|---|---|
| | **Linker with two cleavage sites** | | | |
| FIX | SVSQTSKLT**R**AETVFPDVTQPERGDNNLT**R**IVGGQE | HSA | FIX, FX | 79 |
| FIX | SKLT**R**AETVFPDNNLT**R**IVGGQE | HSA | FIX, FX | 80 |
| FIX | **R**AETVFPDVTQPERGDNNLT**R**IVGGQE | HSA | FIX, FX | 81 |
| FIX | **R**AETVFPERGDNNLT**R**IVGGQE | HSA | FIX, FX | 82 |
| FIX | SVSQTSKLT**R** AETVFPDVDYV NNLT**R** IVGGQE | HSA | FIX, FX | 83 |
| FIX | SVSQTSKLT**R**AETVFPDVD NNLT**R**IVGGQE | HSA | FIX, FX | 84 |
| FIX | SVSQTSKLT**R**AETVFPDVD NNLT**R**IVGGQE | HSA | FIX, FX | 85 |

| FIX | SVSQTSKL T**R**AETVFPDVDYVNSTEAETILDNITQSTQSFN DFTRVVGGEDA | HSA | FIX | 86 |
|---|---|---|---|---|
| FIX | SVSQTSKLT**R**AETVFPDVQSFNDFT**R**VVGGED | HSA | FIX | 87 |
| FIX | SVSQTSKLT**R**AETVFPD VDSFNDFT**R**VVGGED | HSA | FIX | 88 |
| FIX | SVSQTSKLT**R** AETVFPDVNASKPQG**R**IVGGKV | HSA | FIX and FVII | 89 |
| FIX | SVSQTSKLT**R**AETVFPDVNASKPQG**R**LVGGKV | HSA | FIX and FVII | 90 |
| FIX | SVSQTSKLT**R**AETVFPDVNASKPQG**R**TVGGKV | HSA | FIX and FVII | 91 |
| FIX | SVSQTSKLT**R** AETVFPDVD | Fc | | 92 |

Variants and fragments of the described linkers are also encompassed in the present invention as long as the linker can still be cleaved by the protease or the proteases, that cleave the linkers of tables 3a and 3b or by the type of proteases defined above. The term "variants" includes insertions, deletions and substitutions, either conservative or non-conservative.

Other combinations of the cleavage sequences described above and their variants shall be included in the present invention.

In another embodiment, amino acid substitutions are included that change the posttranslational modification pattern of the peptide linker. These can be, for example, substitutions of amino acids that are glycosylated, sulphated, or phosphorylated.

In another embodiment of the invention the peptide linker between the therapeutic polypeptide and the HLEP moiety contains consensus sites for the addition of posttranslational modifications. Preferably such modifications consist of glycosylation sites. More preferably, such modifications consist of at least one N-glycosylation site of the structure Asn - X - Ser/Thr, wherein X denotes any amino acid except proline. Even more preferably such N-glycosylation sites are inserted close to the amino and/or carboxy terminus of the peptide linker such that they are capable of shielding potential neoepitopes which might develop at the sequences where the therapeutic polypeptide moiety is transitioning into the peptide linker or where the peptide linker is transitioning into the albumin moiety sequence.

### Brief Description of the Figures

**Figure 1****:** In vitro activation of FIX-albumin fusion proteins by FXIa at 37°C at a molar ratio of FXIa to fusion protein of about 1:500. One fusion protein with non-cleavable linker (1478/797) and two fusion proteins with cleavable linker (1088/797 and 1089/797) were used. Samples were analyzed by SDS-PAGE under reducing conditions followed by Coomassie blue staining
**Figure 2****:** Pharmakokinetics of activated rec FIX and FIX-albumin fusion proteins with and without cleavable linker in comparison to non-activated fusion proteins.
**Figure 3****:** Inactivation of activated rec FIX or FIX-albumin fusion protein by AT. Residual FIX activity was determined after 120 min using a non-activated partial thromboplastin time assay.

### Examples:

### Example 1: Generation of cDNAs encoding FIX and FIX - albumin fusion proteins

Factor IX coding sequence was amplified by PCR from a human liver cDNA library (ProQuest, Invitrogen) using primers We1403 and We1404 (SEQ ID NO 5 and 6). After a second round of PCR using primers We1405 and We1406 (SEQ ID NO 7 and 8) the resulting fragment was cloned into pCR4TOPO (Invitrogen). From there the FIX cDNA was transferred as an EcoRI Fragment into the EcoRI site of pIRESpuro3 (BD Biosciences) wherein an internal Xhol site had been deleted previously. The resulting plasmid was designated pFIX-496 and was the expression vector for factor IX wild-type.

For the generation of albumin fusion constructs the FIX cDNA was reamplified by PCR under standard conditions using primers We2610 and We2611 (SEQ ID NO 9 and 10) deleting the stop codon and introducing an Xhol site instead. The resulting FIX fragment was digested with restriction endonucleases EcoRI and Xhol and ligated into an EcoRI / BamH1 digested pIRESpuro3 together with one Xhol / BamH1 digested linker fragment as described below.

Two different glycine / serine linker fragments without internal cleavage sites were generated: Oligonucleotides We2148 and We2150 (SEQ ID NO 11 and 12) were annealed in equimolar concentrations (10 pmol) under standard PCR conditions, filled up and amplified using a PCR protocol of a 2 min. initial denaturation at 94°C followed by 7 cycles of 15 sec. of denaturation at 94°C, 15 sec. of annealing at 55°C and 15 sec. of elongation at 72°C, and finalized by an extension step of 5 min at 72°C. The same procedure was performed using oligonucleotides We2156 and We2157 (SEQ ID NO 13 and 14). The resulting linker fragments were digested with restriction endonucleases Xhol and BamH1 and used separately in the above described ligation reaction. The resulting plasmids therefore contained the coding sequence for FIX and a C-terminal extension of a glycine / serine linker.

Two different cleavable linker fragments derived from the activation sites of FIX were generated: Oligonucleotides We2335 and We2336 (SEQ ID NO 15 and 16), containing the activation cleavage site of the FIX light chain / activation peptide border region, were annealed, filled, and amplified as described above. The resulting linker fragment was digested with restriction endonucleases Xhol and BamH1 and used in the above described ligation reaction. The resulting plasmid therefore contained the coding sequence for FIX and a C-terminal extension of a cleavable FIX sequence (amino acids 136 to 154 of SEQ ID NO 2). In a subsequent site directed mutagenesis reaction with a commercially available mutagenesis kit (QuickChange XL Site Directed Mutagenesis Kit, Stratagene) using oligonucleotides We2636 and We2637 (SEQ ID NO 17 and 18) the Xhol site was deleted.

For generation of the second cleavable linker fragment derived from FIX, the same procedure was performed using oligonucleotides We2337 and We2338 (SEQ ID NO 19 and 20) for linker construction. The resulting linker fragment was digested with restriction endonucleases Xhol and BamH1 and used in the above described ligation reaction. The resulting plasmid now contained the coding sequence for FIX and a C-terminal extension of a cleavable FIX sequence derived from the activation cleavage site of the FIX activation peptide / heavy chain border region (amino acids 173 to 186 of SEQ ID NO 2). Oligonucleotides We2638 and We 2639 (SEQ ID NO 21 and 22) were used for deletion of the Xhol site as described above.

In the next cloning step the above generated plasmids were digested with BamH1 and a BamH1 fragment containing the cDNA of mature human albumin was inserted. This fragment had been generated by PCR on an albumin cDNA sequence using primers We1862 and We1902 (SEQ ID NO 23 and 24) under standard conditions.

The final plasmids with non-cleavable glycine/serine linkers were designated pFIX-980 (SEQ ID NO 30) and pFIX-986 (SEQ ID NO 31), respectively. The final plasmids with cleavable linkers derived from FIX sequences were designated pFIX-1088 (SEQ ID NO 40) and pFIX-1089 (SEQ ID NO 49), respectively. Their linker sequences and the C-terminal FIX and N-terminal albumin sequences are outlined below. Proteolytic cleavage sites within the linkers are indicated with arrows, the FIX derived linker sequences are underlined.

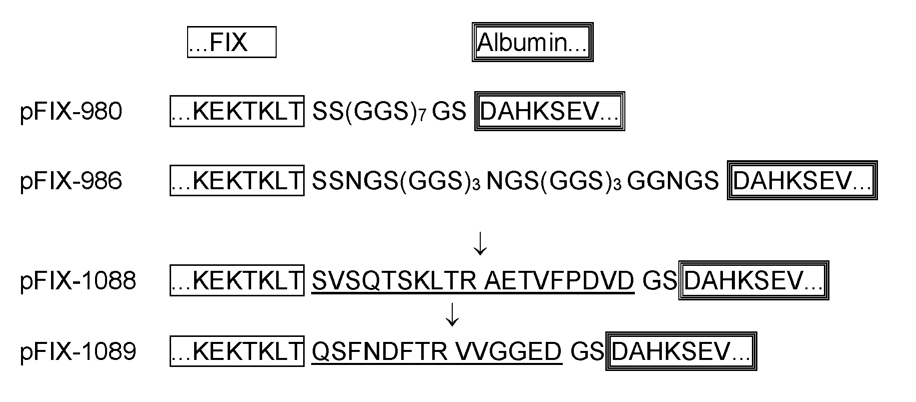

For expression in CHO cells the coding sequences for the FIX albumin fusion protein were transferred into vectors pIRESneo3 (BD Biosciences) or pcDNA3.1 (Invitrogen), respectively.

For efficient processing of the propeptide in cells expressing FIX in high amounts coexpression of furin is required (Wasley LC et al. 1993. PACE/Furin can process the vitamin K-dependent pro-factor IX precursor within the secretory pathway. J. Biol. Chem. 268:8458-8465). Furin was amplified from a liver cDNA library (Ambion) using primers We1791 and We1792 (SEQ ID NO 25 and 26). A second round of PCR using primers We1808 and We1809 (SEQ ID NO 27 and 28) yielded a furin fragment where the carboxyterminal transmembrane domain (TM) was deleted and a stop codon introduced; this fragment was cloned into pCR4TOPO (Invitrogen). From there the furinΔTM cDNA was transferred as an EcoRI/NotI Fragment into the EcoRI/NotI sites of pIRESpuro3 (BD Biosciences) wherein an internal Xhol site had been deleted previously. The resulting plasmid was designated pFu-797. This plasmid was cotransfected with all FIX constructs in a 1:5 (pFu-797 : pFIX-xxx) molar ratio. The amino acid sequence of the secreted furin encoded by pFu-797 is given as SEQ-ID NO 29.

### Example 2: Transfection and expression of FIX and FIX-albumin fusion proteins

Plasmids were grown up in E.coli TOP10 (Invitrogen) and purified using standard protocols (Qiagen). HEK-293 cells were transfected using the Lipofectamine 2000 reagent (Invitrogen) and grown up in serum-free medium (Invitrogen 293 Express) in the presence of 50 ng/ml Vitamin K and 4 µg/ml Puromycin. Transfected cell populations were spread through T-flasks into roller bottles or small-scale fermenters from which supernatants were harvested for purification.
Alternatively, CHO K1 or DG44 cells (Invitrogen) were transfected using the Lipofectamine 2000 reagent (Invitrogen) and grown up in serum-free medium (Invitrogen CD-CHO) in the presence of 50 ng/ml Vitamin K and 500-750 ng/ml Geneticin. High expressing clones were selected and spread through T-flasks into roller bottles or small-scale fermenters from which supernatants were harvested for purification.

### Example 3: Purification of FIX and FIX - albumin fusion proteins

Cell culture harvest containing FIX or FIX albumin fusion protein was applied on a Q-Sepharose FF column previously equilibrated with 50 mM TrisxHCl / 100 mM NaCl buffer pH 8.0. Subsequently, the column was washed with equilibration buffer containing 200 mM NaCl. Elution of the bound FIX or FIX fusion protein was achieved by a salt gradient using 50 mM TrisxHCl / 200 mM NaCl buffer pH 8.0 as a basis. The eluate was further purified by column chromatography on a hydroxylapatite resin. For this purpose, the eluate of the Q-Sepharose FF column was loaded on a hydroxylapatite chromatography column equilibrated with 50 mM TrisxHCl / 100 mM NaCl buffer pH 7.2. The column was washed with the same buffer and FIX or FIX-HSA were eluted using a potassium phosphate gradient at pH 7.2. The eluate was dialyzed to reduce the salt concentration and used for biochemical analysis as well as for determination of the pharmacokinetic parameters. FIX antigen and activity were determined as described in example 5.

### Example 4: Alternative purification scheme of FIX and FIX - albumin fusion proteins

As described in example 3, cell culture harvest containing FIX or FIX albumin fusion protein was purified by chromatography on Q-Sepharose FF. The Q-Sepharose eluate was further purified by chromatography on a Heparin-Fractogel column. For this purpose, the Heparin-Fractogel column was equilibrated using 50 mM Tris x HCl, 50 mM NaCl pH 8.0 buffer (EP), the Q-Sepharose FF eluate was applied and the column was washed with equilibration buffer containing 75 mM NaCl. FIX or FIX albumin fusion protein, respectively, was eluted using EP adjusted to 300 mM NaCl. The Heparin-Fractogel eluate was further purified by chromatography on a hydroxylapatite chromatography column as described in example 3. The purified FIX resp. FIX albumin fusion protein concentrate was subjected to FIX activity and antigen determination according to example 5 and characterized by further in vitro and in vivo investigations.

### Example 5: Determination of FIX activity and antigen

FIX activity was determined as clotting or coagulation activity (FIX:C) using commercially available aPTT reagents (Pathromtin SL and FIX depleted plasma, Dade Behring). An internal substandard calibrated against the WHO International FIX concentrate Standard (96/854) was used as a reference.

FIX antigen (FIX:Ag) was determined by an ELISA acc. to standard protocols known to those skilled in the art. Briefly, microtiter plates were incubated with 100 µL per well of the capture antibody (Paired antibodies for FIX ELISA 1:200, Cedarlane, but other sources of appropriate antibodies may also be applied) overnight at ambient temperature. After washing plates three times with washing buffer B (Sigma P3563), each well was incubated with 200 µL blocking buffer C (Sigma P3688) for one hour at ambient temperature. After another three wash steps with buffer B, serial dilutions of the test sample in buffer B as well as serial dilutions of a substandard (SHP) in buffer B (volumes per well: 100 µL) were incubated for two hours at ambient temperature. After three wash steps with buffer B, 100 µL of a 1:200 dilution of the detection antibody (Paired antibodies for FIX ELISA, peroxidase labelled, Cedarlane) in buffer B were added to each well and incubated for another two hours at ambient temperature. After three wash steps with buffer B, 100 µL of substrate solution (TMB, Dade Behring, OUVF) were added per well and incubated for 30 minutes at ambient temperature in the dark. Addition of 100 µL undiluted stop solution (Dade Behring, OSFA) prepared the samples for reading in a suitable microplate reader at 450 nm wavelength. Concentrations of test samples were then calculated using the standard curve with standard human plasma as reference.

### Example 6: Comparison of FIX-activity/FIX-antigen ratio of different FIX-albumin fusion proteins in cell culture supernatant

Cell culture supernatants of HEK cells transfected with DNA constructs coding for FIX-albumin fusion proteins that contained different linker peptides were subjected to FIX activity and antigen testing as described above (see example 5). The ratio of FIX:C to FIX:Ag was calculated representing a measure directly proportional to molar specific activity of the different constructs.

The results shown in table 4 indicate that there is an increase in activity/antigen ratio upon introduction of cleavable linkers into the FIX-HSA molecule. It also shows that the cleavable linker peptide should have a length of more than two amino acids in order to provide clearly increased activity/antigen ratios.

**Table 4: FIX:C/FIX:Ag ratios of FIX-albumin fusion proteins containing different linker peptides**

| FIX-HSA construct | Linker | FIX:C/FIX:Ag | Fold increase compared to fusion protein 980/797 with non-cleavable linker (GGGGGGV) |
|---|---|---|---|
| 1182/797 | None | < 0.031 | |
| 1366/797 | RI | < 0.068 | |
| 1478/863 | GGGGGGV (Sheffield et al.) | 0.041 | - |
| 980/797 | SS(GGS)₇GS | 0.070 | 1.7 |
| 986/797 | SSNGS(GGS)3NGS (GGS)3GGNGS | 0.076 | 1.9 |
| 1483/863 | SVSQTSKLT**R** AETVFPDVD GSGGS | 0.688 | 16.8 |
| 1088/797 | SVSQTSKLT**R** AETVFPDVD GS | 0.832 | 20.3 |
| 1365/797 | SVSQTSKLT**R** AETVFPDVD | 0.630 | 15.4 |
| 1482/863 | SVSQTSKLT**R** AETVFP | 0.482 | 11.8 |
| 1087/797 | SVSQTSKLT**R** AETVFPDVD GS (FIX deltaKLT) | 0.472 | 11.5 |
| 1089/797 | QSFNDFT**R** VVGGED GS | 0.532 | 13.0 |
| 1091/797 | PERGDNNLT**R** IVGGQE GS | 0.111 | 2.7 |

### Example 7: Comparison of FIX and FIX - albumin fusion proteins in respect to molar specific activity, terminal in vivo half-life and in vivo recovery in rats or rabbits

Purified recombinant wild type FIX (rFIX 496/797) and FIX-albumin fusion proteins (rFIX 980/797, rFIX 986/797, rFIX- 1088/797 and rFIX 1089/797) were tested for FIX activity in a clotting assay as described above. In parallel, the difference of the optical density at 280 and 320 nm was determined as a measure for protein concentration (OD280-320). The ratios of activity per OD280-320 were calculated and based on the molar optical densities the molar specific activities were calculated. In the following table 5 the results are summarized.

**Table 5: Molar specific activities of wt FIX compared to FIX-albumin fusions**

| | Linker | Optical density | FIX clotting activity | Activity/Vol/OD | **Molar specific activity^{±}** |
|---|---|---|---|---|---|
| | | (OD280-320) | (IU/mL) | (IU/mL/OD) | (IU/nmol) |
| | | | | | |
| rFIX, wt (496/797) | | 0,3798 | 21,2 | 55,8 | **4,23** |
| | | | | | |
| rFIX-HSA (non-cleavable, 1478/863 | GGGGGGV (Sheffield et al.) | 2,9189 | 5,8 | 2,0 | **0,23** |
| rFIX-HSA (non-cleavable, 980/797) | SS (GGS)₇ GS | 1,1122 | 3,4 | 3,0 | **0,35** |
| rFIX-HSA (non-cleavable, 986/797) | SS NGS (GGS)3 NGS (GGS)3 GGN GS | 0,8107 | 3,2 | 4,0 | **0,45** |
| rFIX-HSA (cleavable, 1088/797) | FXIa cleavable | 0,3421 | 11,9 | 34,8 | **3,95** |
| rFIX-HSA (cleavable, 1089/797) | FXIa cleavable | 0,4512 | 11,3 | 25,0 | **2,84** |

| | | | | | |
|---|---|---|---|---|---|
| * Molar specific activity based on activity, optical density and the following molar optical densities: Molar optical density of FIX: OD(280nm, 1 mol/L) = 75 810 Molar optical density of albumin: OD(280nm, 1 mol/L) = 37 791 Molar optical density of FIX-albumin fusion protein: OD(280nm, 1 mol/L) = 113 601 | | | | | |

Taking the results summarized in Table 5 into account, it is surprising that two constructs that were generated according to the present disclosure show highly increased molar specific activities compared to the fusion proteins with non-cleavable linkers. In addition, the molar specific activity of these constructs was only moderately decreased compared to wild type rFIX.

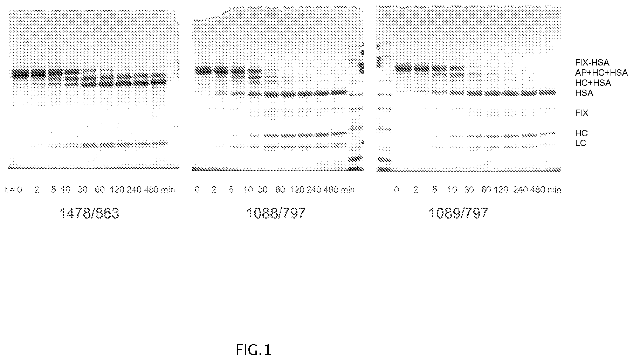

In vitro investigations of the proteolytic cleavage reactions by Factor XIa (FXIa) confirmed that FIX-albumin fusion proteins containing a cleavable linker like e.g. construct no. 1088/797 or 1089/797 are activated and in parallel the linker is cleaved resulting in release of the albumin moiety (Figure 1). The fusion protein with non-cleavable linker did not show a corresponding release of the albumin moiety.

In the case of FVIIa as cleaving protease in the presence of tissue factor, the FIX-albumin fusion proteins 1088/797 or 1089/797 containing a cleavable linker also showed release of the albumin moiety in parallel to release of the FIX activation peptide (Data not shown).

In addition to determination of molar specific coagulation activity, the polypeptides no. 496/797, 980/797, 986/797, 1088/797 and 1089/797 described above were administered intravenously to narcotized CD / Lewis rats (6 rats per substance) and/or rabbits (4 rabbits per substance) with a dose of 50 IU/kg body weight. Blood samples were drawn prior to test substance administration and at appropriate intervals starting at 5 minutes after administration of the test substances. FIX antigen content was subsequently quantified by an ELISA assay specific for human Factor IX (see above). The mean values of the respective groups were used to calculate in vivo recovery after 5 min. Half-lives for each protein were calculated using the time points of the beta phase of elimination (terminal half-life) according to the formula t_{1/2} = In2 / k, whereas k is the slope of the regression line obtained upon plotting FIX:Ag levels in logarithmic scale and time in linear scale.

Calculated in vivo half-lives are summarized in table 6. In rats as well as in rabbits the in vivo half-lives of the FIX-albumin fusion proteins were found to be significantly increased in comparison to non-fused wild-type recombinant FIX prepared inhouse or in comparison to the commercially available recombinant FIX product BeneFIX®. The in vivo half-lives of the albumin fusion proteins compared to BeneFIX® were increased to about 200-400%, depending on the animal species or construct used (Table 6).

To evaluate the in vivo recovery, the FIX antigen levels measured per mL of plasma at their maximum concentrations after intravenous administration (t = 5 min) were related to the amount of product applied per kg. Alternatively, a percentage was calculated by relating the determined antigen level (IU/mL) 5 min post infusion to the theoretical product level expected at 100 % recovery (product applied per kg divided by an assumed plasma volume of 40 mL per kg). The in vivo recoveries (IVR) of the FIX-albumin fusion proteins were significantly higher than the in vitro recoveries of rFIX (496/797) or BeneFIX® (Table 7).

**Table 6:**

| Terminal in vivo half-lives of FIX preparations derived from recombinant expression (BeneFIX®, rFIX 496/797) and FIX albumin fusion proteins (rFIX 980/797, rFIX 986/797, rFIX 1088/797, and rFIX 1089/797) after intravenous administration of 50 IU/kg into rats and/or 50 IU/kg into rabbits, respectively. | | | | |
|---|---|---|---|---|
| | Rat experiments PSR18-05, PSR06-05, PSR02-05 | | Rabbit experiment PSK11-05 | |
| | Terminal half-life (h) | relative to BeneFIX [%] | Terminal half-life (h) | relative to BeneFIX [%] |
| rFIX 496/797 | 4,5* | 91 | n.t. | n.t. |
| rFIX 980/797 | 11,6* | 234 | 36,9° 29,3° (2^{nd} exp.) | 410 326 |
| rFIX 986/797 | 10,5* | 212 | n.t. | n.t. |
| rFIX 1088/797 | 8,3* | 168 | 30,3° | 337 |
| rFIX 1089/797 | 10,5* | 212 | n.t. | n.t. |
| BeneFIX | 4,95* (mean of 5,3 and 4,6) | 100 | 9,0° | 100 |

| | | | | |
|---|---|---|---|---|
| * Determined between 120 and 1440 min ° Determined between 4 and 96 h | | | | |

**Table 7:**

| In vivo recoveries (amount of substance 5 minutes post administration) of recombinant FIX preparations (BeneFIX, rFIX 496/797) and FIX albumin fusion proteins (rFIX 1088/797, rFIX 1089/797) after intravenous administration of 50 IU /kg into rats. The percentage of in vivo recovery was calculated based on an assumed plasma volume of 40 mL/kg. | | |
|---|---|---|
| | rat experiment | |
| | in vivo recovery IU/dL per IU/kg / [%]* | relative to BeneFIX [%] |
| rFIX 496/797 | 0.462/18.5 | 74.6 |
| rFIX 1088/797 | 1.034/41.4 | 166.5 |
| rFIX 1089/797 | 1.063/42.5 | 171.2 |
| BeneFIX | 0.621 / 24.8 | 100 |

| | | |
|---|---|---|
| * Calculated based on a plasma volume of 40 mL/kg | | |

### Example 8: In vitro activation of FIX albumin fusion proteins with/without cleavable linker (1088/797 and 980/797) and determination of pharmacokinetics in rats

FIX-albumin fusion proteins and rec FIX were activated in vitro using commercially available Factor XIa (Kordia). Briefly, identical molar amounts of FIX or FIX-albumin fusion protein (3.0 x 10⁻⁶ mol/L) were activated at 37°C in solution in the presence of FXIa (1.9 x 10⁻⁸ mol/L) and CaCl₂ (1,5 mmol/L) buffered at pH 6.8. After complete activation as shown by SDS-PAGE the reaction was stopped by addition of a 5x molar excess of C1-Inhibitor (Berinert P) based on the amount of FXIa. The samples were stored frozen below -70°C until start of pharmacokinetic investigation.

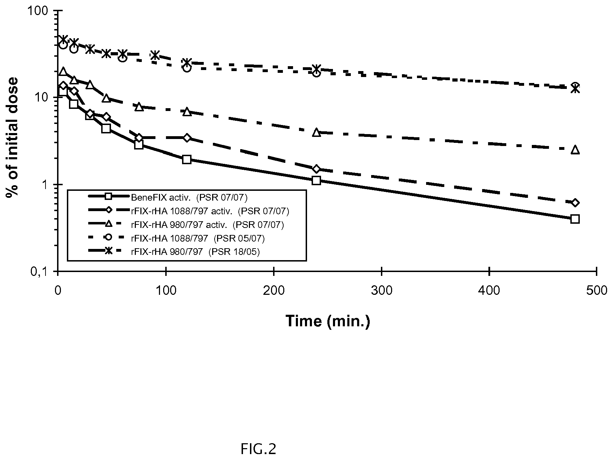

A pharmacokinetic investigation of the activated FIX and the FIX-albumin fusion proteins was performed in rats as described in example 7 and the results were compared to a pharmacokinetic results covering non-activated fusion proteins.
It turned out that the activated fusion proteins demonstrated significantly reduced half-lifes as well as AUC's compared to the non-activated molecules (Figure 2). Upon activation the FIX-fusion protein with cleavable linker (1088/797) showed a pharmacokinetic behaviour very similar to activated rec FIX (BeneFIX) whereas the activated fusion protein with non-cleavable linker (980/797) resulted in a higher initial as well as terminal half-life compared to activated fusion protein 1088/797 with cleavable linker. Therefore, the results clearly demonstrate that the cleavable linker results in increased elimination of the coagulation factor after activation and, therefore, avoids accumulation of potentially thrombogenic, activated fusion proteins with extended half-lives.

### Example 9: Comparison of FIX - albumin fusion proteins with/without cleavable linker in respect to inactivation rate of the activated coagulation factors by antithrombin III (AT)

FIX fusion proteins with (1088/797) and without (980/797) cleavable linker were activated by incubation with FXIa as described in example 8. The activated factors were incubated with AT for 120 min and residual FIXa activity was determined using a manual FIX clotting assay method without activation (naPTT, see below) acc. to Schnitger and Gross. As control samples the activated FIX-albumin fusion proteins were used in presence of the same amount of AT but without incubation.

The F IX activity was determined with the aid of a non-activated partial thromboplastin time assay (naPTT) using FIX deficient plasma from Dade Behring. The samples were prediluted in a buffer of pH 6.8 containing His, Gly, Sucrose, and Tween 80. The whole determination was performed using coagulometers acc. to Schnitger & Gross. A mixture of 0.1 ml F IX deficient plasma, 0.1 ml sample, and 0.1 ml of 0,1 % Phospholipids (Rhone-Poulenc-Nattermann, 1:3 prediluted in imidazole buffer supplemented with 1 % HSA) was incubated for 2 minutes at 37 °C. The coagulation reaction was initiated by adding 0.1 ml 0.025 mol/l CaCl₂ solution and the clotting time was determined.

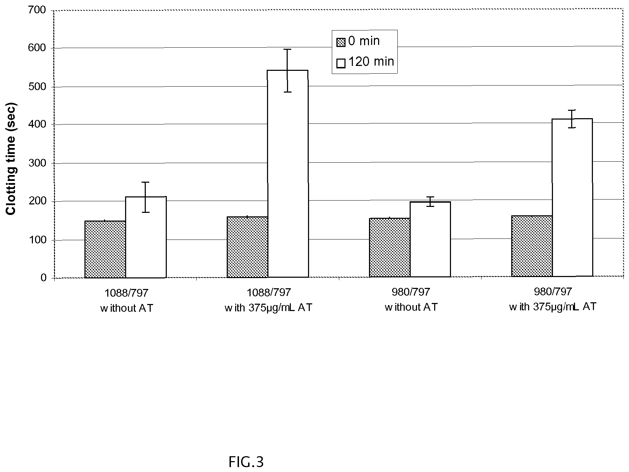

Figure 3 shows the results of a corresponding inactivation experiment. In the case of the fusion protein with cleavable linker (1088/797) an increase in clotting time from 210 to 540 sec (factor of 2.57x) demonstrated an accelerated inactivation process of FIXa activity by AT compared to a fusion protein with non-cleavable linker (980/797) that only showed an increase from 196 to 411 sec (factor of 2.10 x). Most probably, the albumin residue sterically affects the AT dependent inactivation process in the case of the fusion protein with non-cleavable linker whereas in the case of the fusion protein with cleavable linker the albumin residue is cleaved off resulting in an accelerated inactivation by AT.

## Claims

1. Therapeutic fusion protein for use in the treatment or prevention of a blood coagulation disorder comprising
(a) a coagulation factor, wherein the coagulation factor is selected from the list consisting of Factor IX, Factor VIII, van Willebrand Factor, Factor V, Factor X, Factor XI, Factor XII, Factor XIII, Factor I, Factor II (Prothrombin), Protein C, Protein S, GAS6, and Protein Z, ,
(b) a half-life enhancing polypeptide, and
(c) a peptide linker which joins the coagulation factor and the half-life enhancing polypeptide;
wherein the peptide linker is cleavable by proteases involved in coagulation or activated by coagulation enzymes and in that the therapeutic fusion protein has in comparison to the respective therapeutic fusion protein linked by a noncleavable linker having the amino acid sequence GGGGGGV:
(i) an increased molar specific activity in at least one coagulation-related assay; and
(ii) an increased elimination rate of the activated coagulation factor after the peptide linker is proteolytically cleaved in a coagulation-related mode.

2. The therapeutic fusion protein for use according to claim 1 wherein said fusion protein has:
(a) a higher in vivo recovery compared to the in vivo recovery of the respective coagulation factor which is not fused to a half-life enhancing polypeptide; and/or
(b) an increased half-life in plasma compared to the half-life in plasma of the respective coagulation factor which is not fused to a half-life enhancing polypeptide.

3. The therapeutic fusion protein for use according to claims 1 or 2 wherein the coagulation factor is FIX.

4. The therapeutic fusion protein for use according to claims 1 or 2 wherein the coagulation factor is FVIII.

5. The therapeutic fusion protein for use according to claims 1 to 4, wherein the linker is cleavable by:
(a) the protease or proteases, which activate the coagulation factor; and/or
(b) the protease or proteases that are activated upon involvement of the coagulation factor.

6. The therapeutic fusion protein for use according to claims 1 to 5 wherein the linker is cleavable by FXIa and/or FVIIa/TF.

7. The therapeutic fusion protein for use according to claims 1 to 6, wherein the half-life enhancing polypeptide is selected from the group consisting of albumin, polypeptides of the albumin, and immunoglobulins without antigen binding domain.

8. The therapeutic fusion protein for use according to claims 1 to 7, wherein:
(a) the molar specific coagulation-related activity of the therapeutic fusion protein is increased at least 25% compared to that of the therapeutic fusion protein linked by a noncleavable linker having the amino acid sequence GGGGGGV in at least one of the different coagulation-related assays available;
(b) the inactivation rate of the coagulation factor after cleavage of the peptide linker which links the coagulation factor to the half-life enhancing polypeptide is increased by at least 10% as compared to the inactivation rate of the coagulation factor in a corresponding therapeutic fusion linked by a non-cleavable linker having the amino acid sequence GGGGGGV; and/ or
(c) the elimination rate of the coagulation factor after cleavage of the peptide linker which links the coagulation factor to the half-life enhancing polypeptide is increased by at least 10% as compared to the elimination rate of the coagulation factor in a corresponding therapeutic fusion protein linked by a non-cleavable linker having the amino acid sequence GGGGGGV.

9. The therapeutic fusion protein for use according to any of claims 1 to 8 wherein the fusion protein is formulated as a pharmaceutical composition.

10. The therapeutic fusion protein for use according to any of claims 1 to 8, or the pharmaceutical composition for use of claim 9, wherein the blood coagulation disorder is (a) hemophilia B, or (b) hemophilia A.

## Patentansprüche

1. Therapeutisches Fusionsprotein zur Verwendung bei der Behandlung oder Vorbeugung einer Blutgerinnungsstörung, umfassend
(a) einen Gerinnungsfaktor, wobei der Gerinnungsfaktor ausgewählt ist aus der Liste bestehend aus Faktor IX, Faktor VIII, von-Willebrand-Faktor, Faktor V, Faktor X, Faktor XI, Faktor XII, Faktor XIII, Faktor I, Faktor II (Prothrombin), Protein C, Protein S, GAS6 und Protein Z,
(b) ein die Halbwertszeit verbesserndes Polypeptid und
(c) einen Peptidlinker, der den Gerinnungsfaktor mit dem die Halbwertszeit verbessernden Polypeptid verbindet;
wobei der Peptidlinker durch Proteasen, die an der Blutgerinnung beteiligt sind oder durch Blutgerinnungsenzyme aktiviert werden, spaltbar ist und dass das therapeutische Fusionsprotein im Vergleich zu dem entsprechenden therapeutischen Fusionsprotein, das durch einen nichtspaltbaren Linker mit der Aminosäuresequenz GGGGGGV verknüpft ist, Folgendes aufweist:
(i) eine erhöhte molare spezifische Aktivität in wenigstens einem gerinnungsbezogenen Test; und
(ii) eine erhöhte Eliminationsgeschwindigkeit des aktivierten Gerinnungsfaktors nach der gerinnungsbezogenen proteolytischen Spaltung des Peptidlinkers.

2. Therapeutisches Fusionsprotein zur Verwendung nach Anspruch 1, wobei das Fusionsprotein Folgendes aufweist:
(a) eine höhere In-vivo-Wiederfindung verglichen mit der In-vivo-Wiederfindung des entsprechenden Gerinnungsfaktors, der nicht an ein die Halbwertszeit verbesserndes Polypeptid fusioniert ist; und/oder
(b) eine längere Halbwertszeit in Plasma verglichen mit der Halbwertszeit in Plasma des entsprechenden Gerinnungsfaktors, der nicht an ein die Halbwertszeit verbesserndes Polypeptid fusioniert ist.

3. Therapeutisches Fusionsprotein zur Verwendung nach Anspruch 1 oder 2, wobei es sich bei dem Gerinnungsfaktor um FIX handelt.

4. Therapeutisches Fusionsprotein zur Verwendung nach Anspruch 1 oder 2, wobei es sich bei dem Gerinnungsfaktor um FVIII handelt.

5. Therapeutisches Fusionsprotein zur Verwendung nach Ansprüche 1 bis 4, wobei der Linker spaltbar ist durch:
(a) die Protease oder Proteasen, die den Gerinnungsfaktor aktivieren; und/oder
(b) die Protease oder Proteasen, die bei Beteiligung des Gerinnungsfaktors aktiviert werden.

6. Therapeutisches Fusionsprotein zur Verwendung nach Ansprüche 1 bis 5, wobei der Linker durch FXIa und/oder FVIIa/TF spaltbar ist.

7. Therapeutisches Fusionsprotein zur Verwendung nach Ansprüche 1 bis 6, wobei das die Halbwertszeit verbessernde Polypeptid ausgewählt ist aus der Gruppe bestehend aus Albumin, Polypeptiden des Albumins und Immunglobulinen ohne Antigenbindungsdomäne.

8. Therapeutisches Fusionsprotein zur Verwendung nach Ansprüche 1 bis 7, wobei:
(a) die molare spezifische gerinnungsbezogene Aktivität des therapeutischen Fusionsproteins um wenigstens 25% verglichen mit der des therapeutischen Fusionsproteins, das durch einen nichtspaltbaren Linker mit der Aminosäuresequenz GGGGGGV verknüpft ist, in wenigstens einem der zur Verfügung stehenden unterschiedlichen gerinnungsbezogenen Tests erhöht ist;
(b) die Inaktivierungsgeschwindigkeit des Gerinnungsfaktors nach Spaltung des Peptidlinkers, der den Gerinnungsfaktor mit dem die Halbwertszeit verbessernden Polypeptid verknüpft, um wenigstens 10% im Vergleich zur Inaktivierungsgeschwindigkeit des Gerinnungsfaktors in einer entsprechenden therapeutischen Fusion, die durch einen nichtspaltbaren Linker mit der Aminosäuresequenz GGGGGGV verknüpft ist, erhöht ist; und/ oder
(c) die Eliminationsgeschwindigkeit des Gerinnungsfaktors nach Spaltung des Peptidlinkers, der den Gerinnungsfaktor mit dem die Halbwertszeit verbessernden Polypeptid verknüpft, um wenigstens 10% im Vergleich zur Eliminationsgeschwindigkeit des Gerinnungsfaktors in einem entsprechenden therapeutischen Fusionsprotein, das durch einen nichtspaltbaren Linker mit der Aminosäuresequenz GGGGGGV verknüpft ist, erhöht ist.

9. Therapeutisches Fusionsprotein zur Verwendung nach einem der Ansprüche 1 bis 8, formuliert als eine pharmazeutische Zusammensetzung.

10. Therapeutisches Fusionsprotein zur Verwendung nach einem der Ansprüche 1 bis 8 oder pharmazeutische Zusammensetzung zur Verwendung gemäß Anspruch 9, wobei es sich bei der Blutgerinnungsstörung um (a) Hämophilie B oder (b) Hämophilie A handelt.

## Revendications

1. Protéine de fusion thérapeutique pour une utilisation dans le traitement ou la prévention d'un trouble de la coagulation sanguine comprenant
(a) un facteur de coagulation, le facteur de coagulation étant choisi dans la liste constituée par le facteur IX, le facteur VIII, le facteur de van Willebrand, le facteur V, le facteur X, le facteur XI, le facteur XII, le facteur XIII, le facteur I, le facteur II (prothrombine), la protéine C, la protéine S, le GAS6, et la protéine Z,
(b) un polypeptide d'augmentation de la demi-vie, et
(c) un lieur de type peptide qui joint le facteur de coagulation et le polypeptide d'augmentation de la demi-vie ;
le lieur de type peptide étant clivable par des protéases impliquées dans la coagulation ou activées par des enzymes de coagulation et en ce que la protéine de fusion thérapeutique possède en comparaison avec la protéine de fusion thérapeutique respective liée par un lieur non clivable possédant la séquence d'acides aminés GGGGGGV :
(i) une activité spécifique molaire accrue dans au moins une analyse liée à la coagulation ; et
(ii) une vitesse d'élimination accrue du facteur de coagulation activé après que le lieur de type peptide a été clivé de manière protéolytique dans un mode lié à la coagulation.

2. Protéine de fusion thérapeutique pour une utilisation selon la revendication 1, ladite protéine de fusion possédant :
(a) une récupération *in vivo* plus élevée comparée à la récupération *in vivo* du facteur de coagulation respectif qui n'est pas fusionné à un polypeptide d'augmentation de la demi-vie ; et/ou
(b) une demi-vie augmentée dans le plasma comparée à la demi-vie dans le plasma du facteur de coagulation respectif qui n'est pas fusionné à un polypeptide d'augmentation de la demi-vie.

3. Protéine de fusion thérapeutique pour une utilisation selon la revendication 1 ou 2, le facteur de coagulation étant FIX.

4. Protéine de fusion thérapeutique pour une utilisation selon la revendication 1 ou 2, le facteur de coagulation étant FVIII.

5. Protéine de fusion thérapeutique pour une utilisation selon les revendications 1 à 4, le lieur étant clivable par :
(a) la protéase ou les protéases, qui activent le facteur de coagulation ; et/ou
(b) la protéase ou les protéases qui sont activées lors de l'implication du facteur de coagulation.

6. Protéine de fusion thérapeutique pour une utilisation selon les revendications 1 à 5, le lieur étant clivable par FXIa et/ou FVIIa/TF.

7. Protéine de fusion thérapeutique pour une utilisation selon les revendications 1 à 6, le polypeptide d'augmentation de la demi-vie étant choisi dans le groupe constitué par l'albumine, des polypeptides de l'albumine, et des immunoglobulines sans domaine de liaison à un antigène.

8. Protéine de fusion thérapeutique pour une utilisation selon les revendications 1 à 7,
(a) l'activité spécifique molaire liée à la coagulation de la protéine de fusion thérapeutique étant augmentée d'au moins 25 % comparée à celle de la protéine de fusion thérapeutique liée par un lieur non clivable possédant la séquence d'acides aminés GGGGGGV dans au moins l'une des différentes analyses liées à la coagulation disponibles ;
(b) la vitesse d'inactivation du facteur de coagulation après le clivage du lieur de type peptide qui lie le facteur de coagulation au peptide d'augmentation de la demi-vie étant augmentée d'au moins 10 % comparée à la vitesse d'inactivation du facteur de coagulation dans une fusion thérapeutique correspondante liée par un lieur non clivable possédant la séquence d'acides aminés GGGGGGV ; et/ou
(c) la vitesse d'élimination du facteur de coagulation après le clivage du lieur de type peptide qui lie le facteur de coagulation au peptide d'augmentation de la demi-vie étant augmentée d'au moins 10 % comparée à la vitesse d'élimination du facteur de coagulation dans une protéine de fusion thérapeutique correspondante liée par un lieur non clivable possédant la séquence d'acides aminés GGGGGGV.

9. Protéine de fusion thérapeutique pour une utilisation selon l'une quelconque des revendications 1 à 8 formulée en tant que composition pharmaceutique.

10. Protéine de fusion thérapeutique pour une utilisation selon l'une quelconque des revendications 1 à 8, ou composition pharmaceutique pour une utilisation selon la revendication 9, le trouble de la coagulation sanguine étant (a) l'hémophilie B, ou (b) l'hémophilie A.
